(19) Europäisches Patentamt
European Patent Office
Office européen des brevets

(11) EP 3 957 222 A1

(12) EUROPEAN PATENT APPLICATION
published in accordance with Art. 153(4) EPC

(43) Date of publication:
23.02.2022 Bulletin 2022/08

(51) International Patent Classification (IPC):
A47K 17/00 (2006.01)        E03D 9/00 (2006.01)
A61L 2/10 (2006.01)         A61L 9/20 (2006.01)

(21) Application number: 21770927.8

(22) Date of filing: 01.03.2021

(52) Cooperative Patent Classification (CPC):
A47K 17/00; A61L 2/10; A61L 2/24; A61L 2/26;
A61L 9/20; E03D 9/00

(86) International application number:
PCT/JP2021/007746

(87) International publication number:
WO 2021/187073 (23.09.2021 Gazette 2021/38)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.03.2020 JP 2020047045
21.07.2020 JP 2020124574

(71) Applicant: Ushio Denki Kabushiki Kaisha
Tokyo 100-8150 (JP)

(72) Inventors:
• IGARASHI, Tatsushi
Tokyo 100-8150 (JP)
• OHASHI, Hiroyuki
Tokyo 100-8150 (JP)
• OKUMURA, Yoshihiko
Tokyo 100-8150 (JP)
• IMAMURA, Atsushi
Tokyo 100-8150 (JP)

(74) Representative: Tomerius, Isabel
Lang & Tomerius
Patentanwaltspartnerschaft mbB
Rosa-Bavarese-Strasse 5
80639 München (DE)

(54) INACTIVATION DEVICE AND INACTIVATION METHOD

(57) To provide an inactivation apparatus and an inactivation method capable of efficiently inactivating microorganisms and/or viruses, disclosed herein is an inactivation apparatus for inactivating microorganisms and/or viruses, comprising: an ultraviolet light irradiation unit configured to irradiate a surface or a space in a facility or a vehicle that a human or an animal is present with light including ultraviolet light having a wavelength that inactivates the microorganisms and/or viruses; and a controller unit configured to control irradiation and non-irradiation of the light by the ultraviolet light irradiation unit. The ultraviolet light included in the light emitted from the ultraviolet light irradiation unit is light having a wavelength range between 190 nm to 235 nm. The controller unit controls the ultraviolet light irradiation unit to alternately repeat a light-emitting operation and a non-light-emitting operation of the light based on the wavelength of the ultraviolet light included in the light irradiated from the ultraviolet light irradiation unit.

FIG. 12

**Description**

TECHNICAL FIELD

The present invention relates to an inactivation apparatus and method for inactivating harmful microorganisms and/or viruses.

BACKGROUND ART

Facilities where people frequently gather, such as medical facilities, schools, and government offices, and vehicles, such as automobiles, trains, buses, airplanes, and ships, are environments in which harmful microorganisms (bacteria, molds, or the like) and viruses tend to proliferate. These harmful microorganisms and viruses are in particular likely to proliferate in confined or narrow spaces (i.e., enclosed spaces such as hospital rooms, toilet rooms, and inside elevators) in the above facilities.

There is also concern that spaces within the above facilities and/or vehicles impose an increased risk of spreading microbial and/or viral infections.

The above-mentioned harmful microorganisms grow proliferously on surfaces such as a floor and a wall in the above-mentioned spaces or inside humans (or animals, as the case may be) who enter or leave the above-mentioned space, or the harmful microorganisms float in the above-mentioned spaces.

This tendency is in particular pronounced in medical facilities. In other words, infectious microorganisms originating from patients are spread in confined spaces such as hospital rooms for hospitalized patients, toilet rooms inside hospital rooms, and toilet rooms adjacent to outpatient reception areas. The spread infectious microorganisms are most likely to attach to the surfaces (floors, walls, or the like) that constitute the confined space or float in the confined space. For this reason, the infectious microorganisms may infect a next person (e.g., another patient or a visitor) who enters the space (e.g., a toilet room), and in some cases, the infectious disease may even spread in the medical facilities.

Infectious disease may also be spread in the spaces within the above facilities and/or vehicles by infection of others through exhalation or droplets of human (or animal, as the case may be), direct contact with skin or mucous membranes, or contact infection through surfaces such as doorknobs, handrails, switches, or buttons.

In order to resolve such adverse situation described above, measures to decontaminate or sterilize harmful microorganisms (e.g., infectious microorganisms) described above are required in facilities (in particular, medical facilities) where humans (or animals, as the case may be) gather.

Patent Literature 1 (Published Japanese Translation No. 2017-528258 of the PCT International Publication) discloses a decontamination apparatus that irradiates a space to be decontaminated with ultraviolet light (i.e., UV-C light) and decontaminates the space. The decontamination apparatus emits ultraviolet light into the decontamination target space when it is detected that a human is absent in the above decontamination target space.

In addition, Patent Literature 2 (U.S. Patent Application Publication No. 2010/0032859 A) discloses a system in which a human detecting sensor and a door sensor are installed in an elevator, and when those sensors cooperatively detect that there is no human in the elevator and the door is closed, ultraviolet light for sterilization is emitted inside the elevator. The wavelength of the ultraviolet light to be emitted is set to be a wavelength between about 240 nm and about 280 nm.

LISTING OF REFERENCES

PATENT LITERATURE

PATENT LITERATURE 1: Published Japanese Translation No. 2017-528258 of the PCT International Publication

PATENT LITERATURE 2: U.S. Patent Application Publication No. 2010/0032859 A

SUMMARY OF THE INVENTION

PROBLEMS TO BE SOLVED BY THE INVENTION

The proliferating and floating of harmful microorganisms in a space of a facility or a vehicle is often caused by the entry and exit of humans (e.g., patients) or animals having harmful microorganisms into and out of the above confined space. For this reason, decontamination in such a facility is essentially more efficient if it is performed when humans or animals enter or exit the spaces in the facilities or vehicles.

However, irradiation with ultraviolet light having a wavelength suitable for decontamination adversely affects humans or animals. Therefore, in the decontamination system that uses ultraviolet light irradiation for decontamination, as disclosed in Patent Literature 1 (Published Japanese Translation No. 2017-528258 of the PCT International Publication) and Patent Literature 2 (U.S. Patent Application Publication No. 2010/0032859 A) above, in consideration of the safety of humans or animals, the decontamination system is required to be configured to stop emitting the ultraviolet light when humans or animals are present in the area to be irradiated.

Therefore, the conventional decontamination system mentioned above cannot efficiently decontaminate the facilities. In addition, since the surfaces of humans (e.g., patients) or animals cannot be target objects to be decontaminated, it is necessary to broaden the area to be decontaminated over a wide area in consideration of the scope of human (e.g., patient) or animal activities.

The present invention has been made in order to solve the above-mentioned problems and an object thereof is to provide an inactivation apparatus and an inactivation method that are capable of inactivating harmful microorganisms and viruses more efficiently.

SOLUTION TO PROBLEMS

In order to solve the above mentioned problems, according to one aspect of the present invention, there is provided an inactivation apparatus for inactivating microorganisms and/or viruses, comprising: an ultraviolet light irradiation unit configured to irradiate a surface or a space in a facility or a vehicle that a human or an animal is present with light including ultraviolet light having a wavelength that inactivates the microorganisms and/or viruses; and a controller unit configured to control irradiation and non-irradiation of the light by the ultraviolet light irradiation unit, the ultraviolet light included in the light emitted from the ultraviolet light irradiation unit being light having a wavelength range between 190 nm to 235 nm, and the controller unit controlling the ultraviolet light irradiation unit to alternately repeat a light-emitting operation and a non-light-emitting operation of the light based on the wavelength of the ultraviolet light included in the light irradiated from the ultraviolet light irradiation unit.

In this way, by irradiating a surface or a space in a facility or a vehicle in which a human or an animal is present with light including ultraviolet light, it makes it possible to inactivate at least one microorganism and/or virus existing on the surface or the space in the facility or the vehicle, or on the surface of the human or the animal when the human or the animal is present.

In addition, when irradiating with the ultraviolet light since the light-emitting operation and the non-light-emitting operation are alternately repeated, so-called intermittent lighting, it makes it possible to lengthen the period of ultraviolet light irradiation as compared to the case of continuous lighting with the same ultraviolet irradiance. For this reason, the possibility of inactivating microorganisms and/or viruses dispersed in the space can be enhanced, and the usable life of the light source (i.e., the time until the light source needs to be replaced) can be extended as compared to the case of continuous lighting.

Furthermore, since to-be-irradiated light is light having the wavelength range between 190 nm to 235 nm, it makes it possible to inactivate microorganisms and/or viruses while suppressing adverse effects on humans or animals. Also, the conditions for the intermittent lighting are set corresponding to the wavelength of the ultraviolet light to be irradiated. As the degree of influence on the human body due to the ultraviolet light irradiation differs depending on respective wavelengths of the ultraviolet light, by performing the intermittent lighting under the conditions corresponding to the wavelength of the ultraviolet light, it makes it possible to suppress the ultraviolet light from adversely affecting the human body so as to perform decontamination more efficiently.

In the above inactivation apparatus, the ultraviolet light irradiation unit may emit ultraviolet light having a center wavelength between 200 nm and 230 nm, in particular, ultraviolet light having a center wavelength of 222 nm or ultraviolet light having a center wavelength of 207 nm.

In this case, it makes it possible to effectively inhibit photoreactivation of the bacteria. For this reason, even when the light having a wavelength between 300 nm to 500 nm is irradiated in the non-light-emitting operation period subsequent to the light-emitting operation, it makes it possible to prevent the bacteria from being photo-reactivated during the non-light-emitting operation period so that the inactivation effect of the light-emitting operation can be kept maintained. In other words, it makes it possible to achieve an inactivation effect equivalent to that of the continuous lighting.

Furthermore, in the above inactivation apparatus, the controller unit may control the ultraviolet light irradiation unit such that an integrated light intensity by a single light-emitting operation is to be equal to or less than 10 mJ/cm$^2$ or equal to or less than 5 mJ/cm$^2$.

In this case, even when the integrated light intensity per single light-emitting operation is kept low, it makes it possible to achieve a higher inactivation effect. More particularly, even when the amount of ultraviolet light irradiated during one light-emitting operation is lower than the amount of light that can inactivate microorganisms and/or viruses to be inactivated, it makes it possible to appropriately inactivate the microorganisms and/or viruses to be inactivated by repeating the intermittent lighting.

It should be noted that the energy of the light emitted from the light source attenuates in inverse proportion to the square of the distance from the light source. Therefore, the farther the distance from the light source is, the lower the irradiance of the ultraviolet light becomes. Throughout the present disclosure, the term "integrated light intensity" refers to as the amount of irradiation of ultraviolet light to a specific object (e.g., a surface or a space in a facility or a vehicle) for which inactivation of microorganisms and/or viruses thereon is aimed. In other words, it means that there is a region where the integrated light intensity is equal to or less than 10 mJ/cm$^2$, in particular, equal to or less than 5 mJ/cm$^2$, within a region for which the inactivation of the microorganisms and/or viruses is aim.

Yet furthermore, in the above inactivation apparatus, the controller unit may control the ultraviolet light irradiation unit such that a single light-emitting operation time is to be equal to or less than 50% of a sum of the single light-emitting operation time of the ultraviolet light irradiation unit and a single non-light-emitting operation time of the ultraviolet light irradiation unit.

In this case, it makes it possible to extend the time for which the inactivated environment can be kept maintained twice or more with the same integrated light intensity as compared to the case of the continuous lighting.

Yet furthermore, in the above inactivation apparatus, the controller unit may control the ultraviolet light irradiation unit such that a single light-emitting operation time is to be equal to or less than 5% of a sum of the single light-emitting operation time of the ultraviolet light irradiation unit and a single non-light-emitting operation time of the ultraviolet light irradiation unit. In this case, it makes it possible to further extend the time for which the inactivated environment can be kept maintained.

Yet furthermore, in the above inactivation apparatus, the controller unit may control the ultraviolet light irradiation unit such that a single light-emitting operation time is to be equal to or greater than 1% of a sum of the single light-emitting operation time of the ultraviolet light irradiation unit and a single non-light-emitting operation time of the ultraviolet light irradiation unit. In this case, it makes it possible to appropriately maintain the inactivated environment.

Yet furthermore, in the above inactivation apparatus, a single light-emitting operation time of the ultraviolet light irradiation unit is set to a time Ta (sec) satisfying a following formula: $Ta \leq D_{max} / (W*N)$, where a maximum allowable daily ultraviolet light exposure amount to the human body, which is determined based on to the wavelength of the ultraviolet light to be irradiated, is $D_{max}$ (mJ/cm$^2$), irradiance at an ultraviolet irradiated surface of the human body is W (mW/cm$^2$), and a number of times the light-emitting operation is performed per day is N. In this case, it makes it possible to irradiate a human with ultraviolet light having a wavelength suitable for inactivating the microorganisms and/or viruses within a light intensity range that does not adversely affect the human body.

Yet furthermore, in the above inactivation apparatus, a single light-emitting operation time of the ultraviolet light irradiation unit may be set to be equal to or less than one minute.

In this way, even if the single light-emitting operation time is kept short, it makes it possible to achieve a higher inactivation.

Yet furthermore, in the above inactivation apparatus, the ultraviolet light irradiation unit may include a KrCl excimer lamp that emits ultraviolet light having a center wavelength of 222 nm or a KrBr excimer lamp that emits ultraviolet light having a center wavelength of 207 nm.

In this case, it makes it possible to repeat the ultraviolet light-emitting operation time and the pause time at a higher speed by controlling the power supply. For this reason, for example, even when the light-emitting operation time is set to be equal to or less than one minute, still stable light output is obtainable. In addition, by emitting ultraviolet light having a center wavelength of 222 nm or ultraviolet light having a center wavelength of 207 nm, it makes it possible to appropriately suppress the ultraviolet light irradiation from adversely affecting the human body, and also appropriately inhibit the photoreactivation of bacteria.

Yet furthermore, in the above inactivation apparatus, the ultraviolet light irradiation unit may include a light emitting diode (LED) or a laser diode (LD) that emits ultraviolet light. In this case, it makes it possible to repeat the ultraviolet light emitting operation and the pause time at a higher speed by controlling the power supply. Therefore, for example, even when the light-emitting operation time is set to be equal to or less than one minute, still stable light output is obtainable.

Yet furthermore, in the above inactivation apparatus, a single light-emitting operation time of the ultraviolet light irradiation unit may be set to be equal to or less than one hour.

In this case, it makes it possible to appropriately inactivate viruses surviving in the aerosol so as to reduce the risk of infection in the case of a human newly entering the facility or the vehicle.

Yet furthermore, in the above inactivation apparatus, a single light-emitting operation time of the ultraviolet light irradiation unit may be set to be equal to or less than 25 minutes.

In this case, it makes it possible to appropriately perform the ultraviolet light irradiation to small particles of less than 5 μm (i.e., droplet nuclei) which are likely to float in the air before falling on the floor so as to inactivate the airborne viruses and/or bacteria more effectively.

Yet furthermore, in the above inactivation apparatus, a non-light-emitting operation time may be set to be equal to or greater than 2 hours in at least a part of operation cycles of the light-emitting operation and the non-light-emitting operation of the ultraviolet light irradiation unit.

In this way, it makes it possible to appropriately achieve the inactivation effect even when the pause time is set to be longer than the time required for photo-reactivating the bacteria (e.g., approximately 1 to 2 hours in the case of irradiation with ultraviolet light of 254 nm wavelength).

Yet furthermore, in the above inactivation apparatus, the controller unit may control the ultraviolet light irradiation unit to be capable of changing an operation cycle of the light-emitting operation and the non-light-emitting operation in accordance with a proliferation status of the microorganisms and/or viruses in the facility or vehicle.

In this case, it makes it possible to decontaminate a facility or a vehicle more efficiently. In addition, it makes it possible to suppress the ultraviolet light from being unnecessarily irradiated so as to extend the usable life of the light source.

Yet furthermore, according to another aspect of the present invention, there is provided an inactivation method of inactivating microorganisms and/or viruses, comprising the steps of: irradiating, by an ultraviolet light irradiation unit, a surface or a space in a facility or a vehicle that a human or an animal is present with light including ultraviolet light having a wavelength range between 190 nm to 235 nm as light including ultraviolet light having a wavelength that inactivates the microorganisms and/or viruses; and controlling irradiation and non-irradiation of the light by the ultraviolet light irradiation unit such that the ultraviolet light irradiation unit is controlled to alternately repeat a light-emitting operation and a non-light-emitting operation of the light based on the wavelength of the ultraviolet light included in the light irradiated from the ultraviolet light irradiation unit.

In this way, by irradiating a surface or a space in a facility or a vehicle in which a human or an animal is present with light including ultraviolet light, it makes it possible to inactivate at least one microorganism and/or virus existing on the surface or the space in the facility or the vehicle, or on the surface of the human or the animal when the human or the animal is present.

In addition, when irradiating with the ultraviolet light, since the light-emitting operation and the non-light-emitting operation are alternately repeated, so-called intermittent lighting, it makes it possible to lengthen the period of ultraviolet light irradiation as compared to the case of continuous lighting with the same ultraviolet irradiance. For this reason, the possibility of inactivating microorganisms and/or viruses dispersed in the space can be enhanced, and the usable life of the light source (i.e., the time until the light source needs to be replaced) can be extended as compared to the case of continuous lighting.

Furthermore, since to-be-irradiated light is light having the wavelength range between 190 nm to 235 nm, it makes it possible to inactivate microorganisms and/or viruses while suppressing adverse effects on humans or animals. Also, the conditions for the intermittent lighting are set corresponding to the wavelength of the ultraviolet light to be irradiated. As the degree of influence on the human body due to the ultraviolet light irradiation differs depending on respective wavelengths of the ultraviolet light, by performing the intermittent lighting under the conditions corresponding to the wavelength of the ultraviolet light, it makes it possible to suppress the ultraviolet light from adversely affecting the human body so as to perform decontamination more efficiently.

It should be noted that, throughout the present disclosure, the term "inactivation" refers to killing or destroying microorganisms and/or viruses (or eliminating infectivity or toxicity thereof).

ADVANTAGEOUS EFFECT OF THE INVENTION

According to the present invention, it makes it possible to inactivate harmful microorganisms and/or viruses more efficiently by purposively irradiating a human with light including ultraviolet light for a predetermined period of time.

The above mentioned and other not explicitly mentioned objects, aspects and advantages of the present invention will become apparent to those skilled in the art from the following embodiments (detailed description) of the invention by referring to the accompanying drawings and the appended claims.

BRIEF DESCRIPTION OF DRAWINGS

FIG. 1 is a schematic diagram illustrating an exemplary configuration of an inactivation system according to the present embodiments of the present invention.
FIG. 2 is a flowchart exemplarily illustrating a processing procedure according a first embodiment of the present invention.
FIG. 3 is a time chart exemplarily illustrating an operational sequence according to the first embodiment.
FIG. 4 is a flowchart exemplarily illustrating a processing procedure according to a modification to the first embodiment.

FIG. 5 is a time chart exemplarily illustrating an operational sequence according to the modification to the first embodiment.

FIG. 6 is a flowchart exemplarily illustrating a processing procedure according to a second embodiment of the present invention.

FIG. 7 is a time chart exemplarily illustrating an operational sequence according to the second embodiment.

FIG. 8 is a flowchart exemplarily illustrating a processing procedure according to a modification to the second embodiment.

FIG. 9 is a time chart exemplarily illustrating an operational sequence according to the modification to the second embodiment.

FIG. 10 is a time chart exemplarily illustration another operational sequence according to the modification to the second embodiment.

FIG. 11 is a schematic diagram illustrating an exemplary configuration of an inactivation system according to a third embodiment of the present invention.

FIG.12 is a time chart exemplarily illustrating an operational sequence according to the third embodiment.

FIG.13 is a chart illustrating the ultraviolet light absorption spectra of proteins.

FIG. 14 is a chart exemplarily illustrating results of a photoreactivation experiment on bacteria irradiated with ultraviolet light at a wavelength of 254 nm.

FIG. 15 is a chart exemplarily illustrating results of a photoreactivation experiment on bacteria irradiated with ultraviolet light at a wavelength of 222 nm.

FIG. 16 is a chart exemplarily illustrating comparison results between continuous lighting and intermittent lighting at a wavelength of 254 nm.

FIG. 17 is a chart exemplarily illustrating comparison results between continuous lighting and intermittent lighting at a wavelength of 222 nm.

FIG. 18 is a chart illustrating the amount of energy required to inactivate microorganisms and/or viruses.

FIG. 19 is a chart exemplarily illustrating comparison results between continuous lighting and intermittent lighting at a wavelength of 207 nm.

FIG. 20 is a chart illustrating the emission spectrum of a KrCl excimer lamp with a center wavelength of 222 nm.

FIG. 21 is a chart illustrating the emission spectrum of a KrBr excimer lamp with a center wavelength of 207 nm.

FIG. 22A is a time chart of the Operation Example 1.

FIG. 22B is a time chart of the Operation Example 2.

FIG. 23 is a chart exemplarily illustrating experimental results of the Operation Examples 1 and 2.

FIG.24A is a time chart of the Operation Example 3.

FIG.24B is a time chart of the Operation Example 4.

FIG. 25 is a chart exemplarily illustrating experimental results of the Operation Examples 3 and 4.

FIG. 26A is a time chart of the Operation Example 5.

FIG. 26B is a chart exemplarily illustrating experimental results of the Operation Example 5.

## DESCRIPTION OF EMBODIMENTS

Hereinafter, non-limiting embodiments of the present invention will be described in detail with reference to the accompanying drawings.

First Embodiment

The present embodiment will describe an inactivation system that inactivates microorganisms and/or viruses harmful to humans and/or animals by performing ultraviolet light (hereinafter also referred to as "UV light" or simply "UV") irradiation in a particularly confined or narrow space (e.g., an enclosed space such as a hospital room, a toilet room, or inside an elevator) in facilities where people frequently gather. The inactivation system according to the present embodiment is designed to purposively perform the ultraviolet light irradiation on a biological body such as a human (e.g., patient) or an animal for a predetermined period of time, which has been conventionally avoided from the viewpoint of safety so as to inactivate harmful microorganisms and/or viruses.

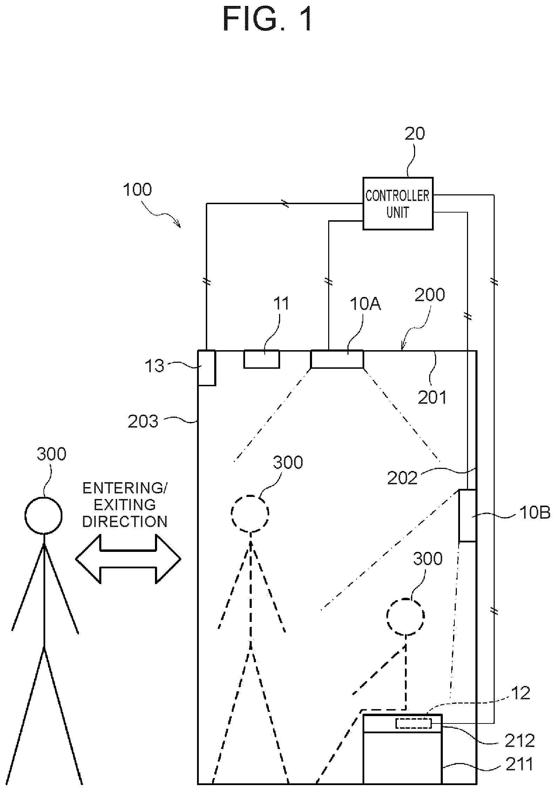

FIG. 1 is a schematic diagram illustrating an exemplary configuration of an inactivation system according to the present embodiment. In the present embodiment pertaining to the inactivation system, an example of an inactivation system that inactivates harmful microorganisms and/or viruses existing in a private toilet room will be described.

The inactivation system includes an inactivation apparatus 100. The inactivation apparatus 100 is provided with at least one of ultraviolet light irradiation units (i.e., UV irradiation units) 10A and 10B that emit ultraviolet light into an enclosed space (e.g., private toilet room) 200. Here, the wavelength range of the ultraviolet light emitted from the UV irradiation units 10A and 10B is, for example, 200 nm to 320 nm.

The UV irradiation unit 10A is disposed on a ceiling 201 in the private toilet room 200. It should be noted that the UV irradiation unit 10A is only required to be provided at the upper side of the private toilet room 200, for example, the UV irradiation unit 10A may be provided at the upper side of the wall portion 202 in the private toilet room 200.

The UV irradiation unit 10A emits the UV light in a downward direction, and the emitted UV light irradiates the space in the private toilet room 200, the wall portion 202, the floor, and the like. In addition, the UV light emitted from the UV irradiation unit 10A are irradiated to a human (e.g., a patient) 300 who has entered the private toilet room 200 from above the human 300 concerned.

On the other hand, The UV irradiation unit 10B is disposed on the wall portion 202 in the private toilet room 200, and the UV irradiation unit 10B emits the UV light mainly in a downward direction from the mounting position thereof. The UV irradiation unit 10B is disposed at a position where the UV light is assumed to be irradiated to a human 300 who takes a predetermined posture at a predetermined position in the private toilet room 200.

More particularly, the UV irradiation unit 10B is installed on the wall portion 202 of the private toilet room 200 on the assumption that the UV irradiation unit 10B irradiates a human 300 with the UV light when the human 300 takes a posture of sitting on the toilet bowl 211. In particular, the UV irradiation unit 10B is installed on the wall portion 202 opposite to the back of the head of a human 300 when the human 300 is seated on the toilet bowl 211.

By installing the UV irradiation unit 10B in this manner, the UV light emitted from the UV irradiation unit 10B is irradiated to a human 300 from above the back of the head side of the human 200 seated on the toilet bowl 211, and is not directly irradiated to the eyes of the human 300.

The inactivation apparatus 100 may also be provided with at least one of a human detecting sensor 11, a pressure sensor 12, and a door sensor 13 as a sensor for detecting the presence or absence of a human 300 in the private toilet room 200.

The human detecting sensor 11 and the door sensor 13 are sensors for detecting the entry and exit of a human into and out of the private toilet room 200, and the pressure sensor 12 is a sensor for detecting the presence of a human at a predetermined position in the private toilet room 200.

The human detecting sensor 11 is installed, for example, on the ceiling 201 as shown in FIG. 1, and detects the presence or absence of a human 300 in the space inside the private toilet room 200.

The pressure sensor 12 is installed, for example, inside the toilet seat 212, as shown in FIG. 1, and detects whether a human 300 is seated on the toilet seat 212 disposed on the toilet bowl 211.

The door sensor 13 is installed, for example, on the door 203, as shown in FIG. 1, and detects opening and closing of the door 203 of the private toilet room 200.

The inactivation apparatus 100 further includes a controller unit 20. The controller unit 20 receives detection signals from each of the sensors 11 to 13, and controls irradiation and non-irradiation of the UV light from the UV irradiation units 10A and 10B based on the detection signals.

More particularly, the controller unit 20 controls the UV irradiation units 10A and 10B to irradiate an inside of the private toilet room 200 including a human 300 with the UV light from at least one of the UV irradiation units 10A and 10B for a predetermined period of time corresponding to the wavelength of the UV light during the period of time when it is determined that the human 300 is present in the private toilet room 200 based on the detection signal of at least one of the sensors 11 to 13.

In the present embodiment, a certain case will be described in which the controller unit 20 controls the irradiation of the UV light from the UV irradiation unit 10A based on the detection signal from the human detecting sensor 11. According to the present embodiment, it is also assumed that, in principle, the irradiation of the UV light from the UV irradiation unit 10A into the private toilet room 200 is continuously performed when a human 300 is absent in the private toilet room 200.

Hereinafter, an operational sequence of the inactivation apparatus 100 according to the present embodiment will be described below.

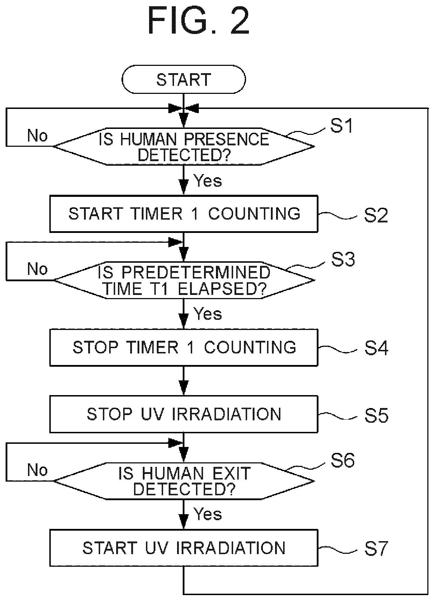

FIG. 2 is a flowchart illustrating an exemplary processing procedure of the inactivation apparatus 100 according to the present embodiment.

First, in step S1, the controller unit 20 determines whether or not the presence of a human 300 in the private toilet room 200 has been detected based on the detection signal from the human detecting sensor 11. When the controller unit 20 determines that the presence of a human 300 have not yet been detected, the controller unit 20 waits until the presence of a human 300 is detected. On the other hand, when the controller unit 20 determines that the presence of a human 300 is detected, the processing proceeds to step S2.

In step S2, the controller unit 20 starts counting a timer 1, which serves as a counter.

Subsequently, in step S3, the controller unit 20 determines, based on a count value of the timer 1, whether the predetermined time T1 has elapsed since the timer 1 started counting, in other words, whether the predetermined time T1 has elapsed since the presence of a human 300 in the private toilet room 200 was detected. When the predetermined time T1 has not yet elapsed, the controller unit 20 waits until the predetermined time T1 has elapsed. On the other hand, when the controller unit 20 determines that the predetermined time T1 has elapsed, the processing proceeds to step S4.

Here, the predetermined time T1 is set to a time corresponding to the wavelength of the UV light emitted from the UV irradiation unit 10A, and is set to a time equal to or less than the maximum time that is allowable to irradiate a biological body with a particular wavelength of UV light in accordance with the safety standard. Details of the predetermined time T1 will be described later.

In step S4, the controller unit 20 terminates the counting of the timer 1 and resets the count value of the timer 1.

In step S5, the controller unit 20 stops the emission of the UV light from the UV irradiation unit 10A.

In step S6, the controller unit 20 determines whether or not the human 300 has exited from the private toilet room 200 based on the detection signal from the human detecting sensor 11. When the controller unit 20 determines that the human 300 has not yet exited therefrom, the controller unit 20 waits as it is. On the other hand, when the controller unit 20 determines that the human 300 has exited therefrom, the processing proceeds to step S7.

In step S7, the controller unit 20 starts the emission of the UV light from the UV irradiation unit 10A and the processing returns to step S1.

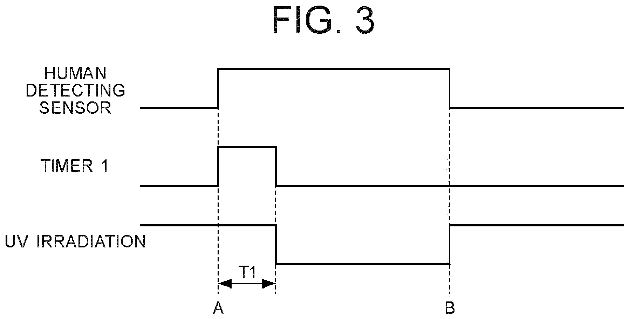

FIG. 3 is a time chart illustrating an exemplary operational sequence of the inactivation apparatus 100 according to the present embodiment.

Prior to the entry of a human 300 into the private toilet room 200, in other words, while a human 300 is absent in the private toilet room 200, the irradiation of the UV light from the UV irradiation unit 10A into the private toilet room 200 is being continuously performed.

When a human 300 enters the private toilet room 200 from this state, the presence of the human 300 is detected by the human detecting sensor 11 at the time point A, and the timer 1 starts counting. Subsequently, after a predetermined time T1 elapses from the time point A, the irradiation of the UV light into the private toilet room 200 and to the human 300 is stopped.

As described above, even after the human 300 enters the private toilet room 200, the UV light is emitted from the UV irradiation unit 10A into the private toilet room 200 until the predetermined time T1 elapses, and thus the emitted UV light is also irradiated to the human 300 in the private toilet room 200.

Thereafter, when the human 300 exits from the private toilet room 200, at the time point B, it is detected that the human has exited therefrom by the human detecting sensor 11, and the controller unit 20 resumes the irradiation of the UV light into the private toilet room 200.

Hereinafter, the irradiation time (i.e., the predetermined time T1) for irradiating a human 300 with the UV light will be described below.

The UV light having the wavelength range of 200 nm to 320 nm emitted from the UV irradiation units 10A and 10B includes the UV light that adversely affects the human body. For example, when being irradiated with the UV light within the above wavelength range, cancers may be induced due to erythema or DNA damage of the skin, or eye disorders (such as ocular pain, hyperemia, cornea inflammation) may occur.

However, it should be noted that the irradiation of the UV light within the above wavelength range does not adversely affect the biological body unless the integrated light intensity (in other words, cumulative light amount or dose amount) to the biological body, which is a target object to be irradiated, exceeds a predetermined light amount. Focusing on those findings, the present inventors set the irradiation time (i.e., the predetermined time T1) for humans, and conceived an inactivation apparatus that purposively irradiates humans with the UV light.

The UV light exposure amount per day to a human using an enclosed space (e.g., private toilet room) is assumed to be D ($mJ/cm^2$). Where the irradiance on the UV exposed surface of a human is W ($mW/cm^2$), the number of times a human enters an enclosed space (e.g., private toilet room) per day is N, and the irradiation time of the UV light during one stay in a private toilet room is T1, the UV light exposure amount per day D is expressed as the following formula 1.

$$D \ (mJ/cm^2) = W \ (mW/cm^2) * N \ (times) * T1 \ (sec) \qquad \dots (1)$$

Then the maximum allowable UV light exposure amount per day to a human using the enclosed space (e.g., private toilet room) is assumed to be $D_{max}$ ($mJ/cm^2$), $D_{max} \geq D$ is considered to be sufficient to prevent adverse effects on humans caused by the UV light irradiation.

In other words, the UV light irradiation time T1 during one stay in a private toilet room is expressed as the following formula 2.

$$T1 \leq D_{max} / (W * N) \qquad \dots (2)$$

For example, consider the case in which a low-pressure mercury lamp emitting UV light having a wavelength of 253.7 nm is used as the UV light source. In this case, the maximum allowable daily UV light exposure amount for the UV light having the wavelength of 253.7 nm is $D_{max} = 6$ ($mJ/cm^2$) according to the safety standard. This value is defined by ACGIH (American Conference of Governmental Industrial Hygien-ists) and JIS Z 8812 (Measurement method of harmful ultraviolet radiation). It should be noted that the numerical value of the maximum allowable daily UV light exposure amount $D_{max}$ in the present disclosure is no more than the current value, which may be changed in the future.

Assuming that the irradiance on the UV irradiated surface of a human to be irradiated with the UV light is 0.022 (mW/cm$^2$) and the number of times the private toilet room belonging to a hospital room is used (i.e., the number of times entering the private toilet room) N is 10 times per day, the above formula (2) indicates that the UV light irradiation time T1 during one stay in the private toilet room is 30 seconds or less.

In other words, when the UV light source provided in the UV irradiation unit 10A is a low pressure mercury lamp that emits the UV light having a wavelength of 253.7 nm, as long as the predetermined time T1 set in FIGS. 2 and 3 is 30 seconds or less, the UV light irradiation by the low pressure mercury lamp is considered not to adversely affect a human 300. Therefore, in this case, the predetermined time T1 is set, for example, to 30 seconds, which is the maximum time.

It should be noted that the above mentioned irradiance on the UV irradiated surface of a human to be irradiated with the UV light is calculated by assuming that the head (e.g., top) of a human 300 standing in the enclosed space (e.g., private toilet room) 200 is the UV irradiated surface and that the distance from the ceiling 201 of the enclosed space (e.g., private toilet room) 200 to the head of the human 300 standing on the floor is the UV light irradiation distance.

In the above example, the number of times N that a human enters the above enclosed space (e.g., private toilet room) is defined as the number of times the private toilet room belonging to the hospital room is used per day, and N = 10 times. However, in the case of a private toilet room adjacent to an outpatient department area of a hospital, the number of times N' that a plurality of humans waiting in the waiting area respectively use the private toilet room per day is considered to be less than the number of times N that the private toilet room belonging to the hospital room is used. Therefore, in this case, for example, N'=2 to 3 times, and the UV light irradiation time T1 may be set based on N'.

It is preferable to set the number of times N that a human enters the enclosed space per day to a larger number on the safety side.

Furthermore, low pressure mercury lamps do not light up immediately after being supplied with power but require some time to light up. Therefore, when a low pressure mercury lamp is used as the UV light source, it is not possible to alternately repeat irradiation and non-irradiation of the UV light at a relatively short interval by controlling the power supply. To cope with this shortcoming, in this case, a shutter for light shielding may be provided, and irradiation and non-irradiation of the UV light may be controlled by controlling the opening and closing of the shutter while the low pressure mercury lamp is kept turned on.

Alternatively, for example, a KrCl excimer lamp that emits UV light having a center wavelength of 222 nm may be used as the UV light source.

In the case of excimer lamps, the excimer lamps light up immediately after being supplied with power. Therefore, unlike the case in which the low pressure mercury lamp is used as the UV light source, there is no need to provide a shutter for light shielding. In other words, when alternately repeating the irradiation and non-irradiation of the UV light at a relatively short interval, it is sufficient to control the power supply to the excimer lamp.

More importantly, the UV light having a center wavelength of 222 nm is light that kills bacteria and other organisms but has little adverse effect on human cells.

The shorter the wavelengths of UV radiation, the smaller the penetration power (i.e., transmissivity). For example, the UV radiation with a short wavelength of about 200 nm passes through water very efficiently, but is largely absorbed by the outer part (i.e., cytoplasm) of human cells. For this reason, such short wavelength UV radiation at about 200 nm may not have sufficient energy to reach the cell nuclei, which contain DNAs sensitive to radiation. Therefore, UV radiation at the shorter wavelengths described above has typically less adverse effects to human cells, in other words, less adverse effects on humans.

On the other hand, bacteria are typically much smaller physically than human cells. More particularly, a typical bacterial cell is less than about 1 $\mu$m in diameter, whereas a human cell is typically about 10 $\mu$m to 30 $\mu$m in diameter, depending on the type and location thereof.

Therefore, the above described UV radiation at the shorter wavelength can easily penetrate and sterilize the bacteria.

According to the current safety standard, the maximum allowable daily UV light exposure amount of the UV light having a wavelength of 222 nm is $D_{max} = 22$ (mJ/cm$^2$), which is larger than the UV light having a wavelength of 253.7 nm described above. In other words, it is understood from the safety standard that the UV light having the wavelength of 222 nm has less adverse effects on humans than the UV light having the wavelength of 253.7 nm.

As in the case of using a low pressure mercury lamp emitting the UV light having the wavelength of 253.7 nm described above, assuming that the irradiance on the UV irradiated surface of a human to be irradiated with the UV light is 0.022 (mW/cm$^2$) and the number of times the private toilet room belonging to the hospital room is used (i.e., the number of times entering the private toilet room) N is 10 times per day, the above formula (2) indicates that, when the KrCl excimer lamp emitting the UV light having a center wavelength of 222 nm is used, the UV light irradiation time T1 during one stay in the private toilet room is 100 seconds or less.

Thus, when the UV light source provided in the UV irradiation unit 10A is the KrCl excimer lamp emitting the UV light having a center wavelength of 222 nm, the predetermined time T1 set in FIGS. 2 and 3 can be set, for example, to 100 seconds, which is the maximum time.

It should be noted that, although the KrCl excimer lamp emits light having the center wavelength of 222 nm, the KrCl excimer lamp also emits a small amount of light in other wavelength ranges. Therefore, in the case of actual use, it is preferable to use a wavelength selective filter that transmits only the light within the wavelength range of 190 nm to 235 nm, which has little adverse effects on the human body, and cuts the light in other wavelength ranges.

As the wavelength selective filter, for example, an optical filter having a dielectric multilayer film made of $HfO_2$ and $SiO_2$ layers can be used. More particularly, the optical filter may have a structure in which a dielectric multilayer film formed by alternately laminating $HfO_2$ layers and $SiO_2$ layers on one surface of a substrate made of synthetic fused silica glass, and an AR coating of the $HfO_2$ layers and $SiO_2$ layers is applied to the other surface of the substrate. For example, the thickness of the $HfO_2$ layers in the dielectric multilayer film may be approximately 240 nm, the thickness of the $SiO_2$ layers therein may be approximately 1460 nm, respectively, and the total number of layers of the $HfO_2$ and $SiO_2$ layers may be 33 layers.

Alternatively, an optical filter having a dielectric multilayer of $SiO_2$ layers and $Al_2O_3$ layers may be used as the wavelength selective filter.

However, when using the optical filter having the dielectric multilayer made of $HfO_2$ layers and $SiO_2$ layers as the wavelength selective filter, it makes it possible to reduce the total number of layers as compared to the case using an optical filter having the dielectric multilayer made of $SiO_2$ layers and $Al_2O_3$ layers. As a result, it makes it possible to increase the transmittance of the UV light at an incident angle of 0°, and to ensure the light intensity of the UV light in the desired wavelength range of 190 to 235 nm. In addition, by reducing the total number of layers, the cost of the layers can be reduced accordingly.

As described above, the inactivation apparatus 100 according to the present embodiment includes the ultraviolet light irradiation unit (i.e., UV irradiation unit) 10A that irradiates the inside of the enclosed space (e.g., private toilet room 200) in which a person can enter and exit with light including the UV light having a wavelength that inactivates microorganisms and/or viruses harmful to the human body. The inactivation apparatus 100 also includes the human detecting sensor 11 that detects the presence or absence of a human in the private toilet room 200 as a sensor for detecting the whereabouts (i.e., presence or absence) of a human in the private toilet room 200. The controller unit 20 controls the UV irradiation unit 10A to irradiate the space including the human for a predetermined time (T1) corresponding to the wavelength of the UV light emitted from the UV irradiation unit 10A within a period of time when it is determined that the human is present in the private toilet room 200 based on the detection signal from the human detecting sensor 11.

In this manner, by purposively irradiating a human with light including the UV light for a predetermined time T1, it makes it possible to inactivate at least one harmful microorganism or virus existing on the surface of the human body (e.g., the surface of the skin or clothing). Therefore, it makes it possible to suppress the harmful microorganism or virus from being dispersed from the person into the enclosed space (e.g., the private toilet room 200).

In addition, the harmful microorganisms and/or viruses attaching to the surface of the skin or clothing are reduced or eliminated from the human who has exited from the enclosed space (e.g., private toilet room 200), as the human has been already irradiated with the UV light. Therefore, it makes it possible to suppress the harmful microorganisms and/or viruses from being dispersed from the human who has exited from the enclosed space (e.g., private toilet room 200) to other outer areas. As a result, it makes it possible to suppress the area to be decontaminated from expanding in the facility so that the facility can be decontaminated more efficiently.

Furthermore, the predetermined time T1 for irradiating a human with the UV light may be a time corresponding to the wavelength of the UV light concerned. The degree of influence on the human body by the UV light irradiation differs depending on the wavelength of the UV light concerned. Therefore, by setting the predetermined time T1 in accordance with the wavelength of the UV light, it makes it possible to appropriately irradiate a human with UV light having a wavelength suitable for decontamination within a light intensity (i.e., light amount) range that does not adversely affect the human body.

More particularly, the predetermined time T1 is set to the time T1 that satisfies the above formula (2). Thus, since the UV light irradiation time is set for each wavelength of the UV light to be irradiated in accordance with the safety standard, it makes it possible to appropriately suppress the adverse effects on the human body due to the UV light irradiation.

It should be noted that the controller unit 20 may acquire information on the wavelength of the UV light emitted from the UV irradiation unit 10A, set the predetermined time T1 in accordance with the safety standard based on the acquired information so as to control the irradiation and non-irradiation of the UV light by the UV irradiation unit 10A. In other words, the predetermined time T1 may be variably set according to the light source to be used.

In addition, according to the present embodiment, the controller unit 20 can control the UV irradiation unit 10A to irradiate the space including a human with the UV light for a predetermined time T1 after detecting that the human has entered the private toilet room 200 by the human detecting sensor 11. In this manner, immediately after the human enters the private toilet room 200, the human can be irradiated with the UV light. As a result, it makes it possible to suppress the harmful microorganisms and/or viruses from being dispersed from the human into the private toilet room 200 more efficiently.

Furthermore, according to the present embodiment, the controller unit 20 may control the UV irradiation unit 10A to irradiate the inside of the private toilet room 200 in which no human is present (i.e., the private toilet room 200 from which the human has exited) with the UV light at the time when the human detecting sensor 11 determines that no human is present in the private toilet room 200 (i.e., the human has exited from the private toilet room 200).

In this manner, by irradiating the inside of the private toilet room 200 in which no human is present with the UV light, it makes it possible to inactivate at least a part of the harmful microorganisms and/or viruses that originally existed in the private toilet room 200, the harmful microorganisms and/or viruses that are dispersed inside the private toilet room 200 due to the entry of a human, and the harmful microorganisms and/or viruses that are floating in the air flowing into the private toilet room 200 when a human enters the private toilet room 200. In addition, when a human is absent in the private toilet room 200, the above inactivation can be performed more effectively by continuously irradiating the inside of the private toilet room 200 with the UV light.

Yet furthermore, the UV irradiation unit 10A may be disposed at a position emitting light downwardly from the upper side of the private toilet room 200, more particularly at the ceiling 201 of the private toilet room 200. Therefore, the UV irradiation unit 10A may irradiate the entire private toilet room 200 with light including the UV light. As a result, it makes it possible to appropriately inactivate, for example, harmful microorganisms and/or viruses attaching to the wall portion 202, the door 203, the floor, and the like of the private toilet room 200 entirely.

It should be noted that, in the present embodiment described above, a certain case has been described in which the human detecting sensor 11 that detects the presence or absence of a human in the private toilet room 200 is used as a sensor for detecting the entry and exit of a human into the private toilet room 200. However, the present embodiment is not limited thereto, and any sensor can be used as long as it is capable of detecting the entry of a human into the private toilet room 200 and the exit of a human from the private toilet room 200.

(Modification to First Embodiment)

In the first embodiment described above, a certain case has been described in which the UV light irradiation from the UV irradiation unit 10A to the inside of the private toilet room 200 is continuously performed when a human 300 is absent in the private toilet room 200. However, when a human is absent in the private toilet room 200, the UV light irradiation to the inside of the private toilet room 200 may be performed for a predetermined time T2.

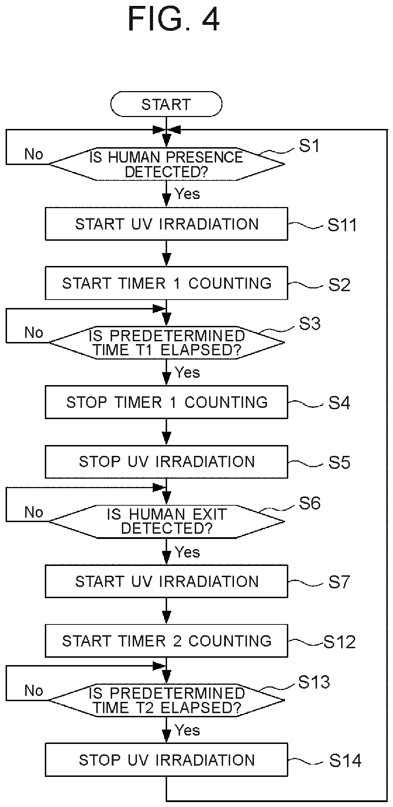

FIG. 4 is a flowchart illustrating an exemplary processing procedure performed by the inactivation apparatus 100 according to the present modification. Referring to FIG. 4, the same step numbers are assigned to the portions that perform the same processing as in FIG. 2, and the following description will focus on the portions that differ in processing.

In step S1, when the controller unit 20 detects the presence of a human 300 in the private toilet room 200, the processing proceeds to step S11 and the controller unit 20 controls the UV irradiation unit 10A to start emitting the UV light and the processing proceeds to step S2.

Subsequently in step S7, the UV irradiation unit 10A starts emitting the UV light, and the processing proceeds to step S12. In step S12, the controller unit 20 starts counting the timer 2, which is another counter.

Subsequently in step S13, the controller unit 20 determines, based on the count value of the timer 2, whether or not a predetermined time T2 has elapsed since the time 2 starts counting, in other words, whether or not the predetermined time T2 has elapsed since the human 300 exited from the private toilet room 200. When the predetermined time T2 has not yet elapsed, the controller unit 20 waits until the predetermined time T2 has elapsed. On the other hand, when the controller unit 20 determines that the predetermined time T2 has elapsed, the processing proceeds to step S14.

Here, the predetermined time T2 is set to a time that is sufficient to inactivate at least a part of the harmful microorganisms and/or viruses existing in the private toilet room 200 from which the human 300 has left.

In step S14, the controller unit 20 controls the UV irradiation unit 10A to stop emission of the UV light and the processing returns to step S1.

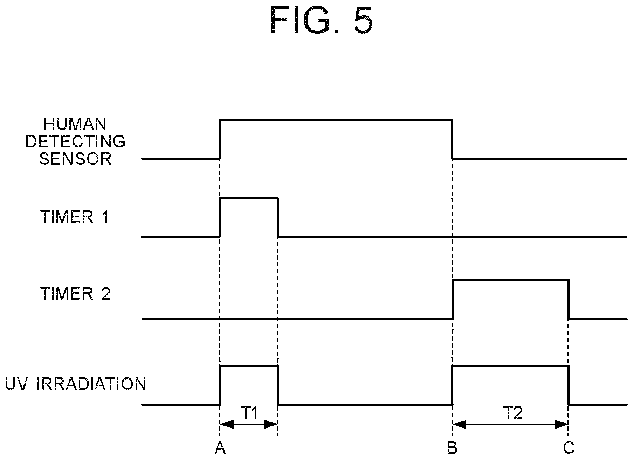

FIG. 5 is a time chart illustrating an exemplary operational sequence performed by the inactivation apparatus 100 according to the present modification.

Here, it is assumed that the UV light irradiation from the UV irradiation unit 10A to the inside of the private toilet room 200 is stopped before a human 300 enters the private toilet room 200.

When a human 300 enters the private toilet room 200 in this state, at the time point A, the human detecting sensor 11 detects the presence of the human 300, the timer 1 starts counting, and the UV irradiation unit 10A starts the irradiation of the UV light to the inside of the private toilet room 200 and the human 300. Subsequently, after a predetermined time T1 elapses from the time point A, the UV irradiation unit 10A stops the irradiation of the UV light to the inside of the private toilet room 200 and the human 300.

As described above, even when the UV irradiation unit 10A stops the UV light irradiation before a human 300 enters the private toilet room 200, the UV irradiation unit 10A starts the UV light irradiation once a human 300 enters the private toilet room 200. Then, the UV irradiation unit 10A irradiates the human 300 in the private toilet room 200 with the UV light until the predetermined time T1 elapses from that time point.

Thereafter, when the human 300 exits from the private toilet room 200, at the time point B, the human detecting sensor 11 detects that the human 300 has exited therefrom, the timer 2 starts counting, and the controller unit 20 resumes the irradiation of the UV light into the private toilet room 200.

When a predetermined time T2 elapses from the time point B when the human 300 exits from the private toilet room 200, the irradiation of the UV light into the private toilet room 200 stops at the time point C.

As described above, when the controller unit 20 determines via the human detecting sensor 11 that no human is present in the private toilet room 200, the UV irradiation unit 10A may irradiate the inside of the private toilet room 200 in which no human is present with the UV light, and subsequently stop the UV light irradiation after the UV light irradiation is performed for a certain period of time (i.e., predetermined time T2). By allowing the UV light irradiation to be performed to the inside of the private toilet room 200 in which no human is present for a certain period of time, it makes it possible to provide a pause time for the UV light source provided in the UV irradiation unit 10A, thereby extending the usable life of the UV light source.

Second Embodiment

Hereinafter, a second embodiment according to the present invention will be described.

In the first embodiment described above, a certain case has been described in which the human detecting sensor 11 detects that a human 300 has entered the enclosed space (e.g., private toilet room) 200 and the controller unit 20 controls the irradiation of the UV light from the UV irradiation unit 10A based on the detection signal from the human detecting sensor 11. In the second embodiment, a case will be described in which the pressure sensor 12 detects that a human 300 is seated on the toilet seat 212 in the private toilet room 200, and the controller unit 20 controls the irradiation of the UV light based on the detection signal from the pressure sensor 12.

According to the present embodiment, when a human 300 is absent in the private toilet room 200, in principle, the UV light irradiation to the inside of the private toilet room 200 is assumed to be continuously performed.

Also, the UV light irradiation is assumed to be performed using the UV irradiation unit 10B.

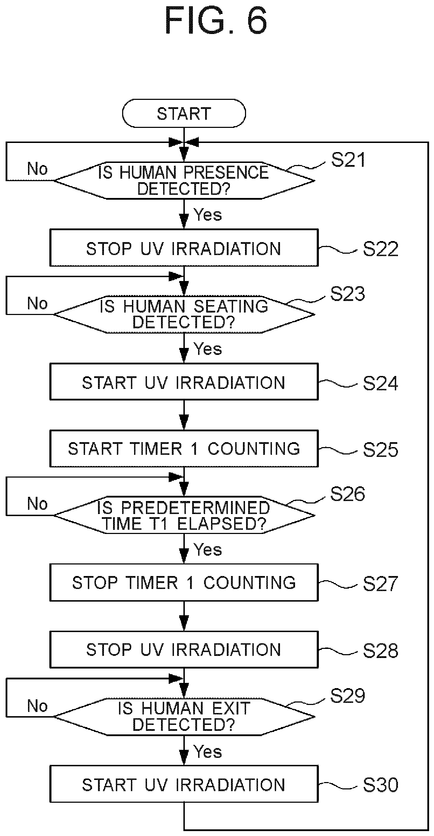

FIG. 6 is a flowchart illustrating an exemplary processing procedure performed by the inactivation apparatus 100 according to the present embodiment.

First, in step S21, the controller unit 20 determines whether or not the presence of a human 300 in the private toilet room 200 has been detected based on the detection signal from the human detecting sensor 11. When the controller unit 20 determines that the presence of a human 300 have been not yet detected, the controller unit 20 waits until the presence of a human 300 are detected. On the other hand, when the controller unit determines that the presence of a human 300 are detected, the processing proceeds to step S22.

In step S22, the controller unit 20 controls the UV irradiation unit 10B to stop emitting the UV light and the processing proceeds to step S23.

In step S23, the controller unit 20 determines, based on the detection signal from the pressure sensor 12, whether or not the seating of a human 300 on the toilet seat 212 has been detected. When the controller unit 20 determines that the seating of a human 300 has not been detected, the controller unit 20 waits until the seating is detected. On the other hand, when the seating of the human 300 is detected, the processing proceeds to step S24.

In step S24, the controller unit 20 controls the UV irradiation unit 10B to start emitting the UV light, and the processing proceeds to step S25.

In step S25, the controller unit 20 starts counting the timer 1, which is a counter.

Subsequently in step S26, the control unit 20 determines, based on the countvalue of the timer 1, whether or not the predetermined time T1 has elapsed since the timer 1 started counting, in other words, whether or not the predetermined time T1 has elapsed since the seating of a human 300 on the toilet seat 212 has been detected. When the predetermined time T1 has not elapsed, the controller unit 20 waits until the predetermined time T1 has elapsed. On the other hand, when the controller unit 20 determines that the predetermined time T1 has elapsed, the processing proceeds to step S27.

Here, the predetermined time T1 is a time corresponding to the wavelength of the UV light emitted from the UV irradiation unit 10B, and is set to be equal to or less than the maximum time that is allowable to irradiate a biological body with the UV light concerned in accordance with the safety standard. The predetermined time T1 can be, for example, the same time as in the first embodiment.

The UV irradiation unit 10B may be installed on the wall portion 202 of the private toilet room 200 on the assumption that the UV irradiation unit 10B emits the UV light from above the back of the head side of a human 300 when the human 300 is in the posture of sitting on the toilet bowl 211. Therefore, the irradiance on the UV irradiated surface (e.g., head) of the human 30 in this case may be the same value as the irradiance in the case of irradiating the standing human 300 with the UV light using the UV irradiation unit 10A as in the first embodiment described above. In other words, the irradiance on the UV irradiated surface of a human to be irradiated with the UV light may be 0.092 $(mW/cm^2)$.

In step S27, the controller unit 20 terminates counting the timer 1 and resets the count value of the timer 1.

In step S28, the controller unit 20 controls the UV irradiation unit 10B to stop emitting the UV light.

In step S29, the controller unit 20 determines whether or not the human 300 has exited from the private toilet room 200 based on the detection signal from the human detecting sensor 11. When the controller unit 20 determines that the human 300 has not yet exited therefrom, the controller unit 20 waits as it is. On the other hand, when the controller unit 20 determines that the human 300 has exited therefrom, the processing proceeds to the step S30.

In step S30, the controller unit 20 controls the UV irradiation unit 10B to start emitting the UV light and the processing returns to step S21.

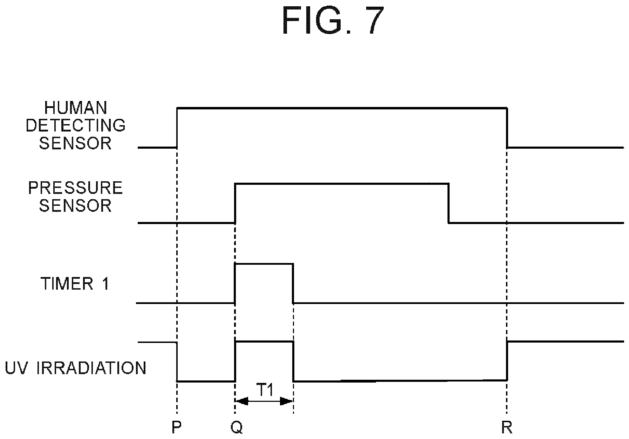

FIG. 7 is a time chart illustrating an exemplary operational sequence performed by the inactivation apparatus 100 according to the present embodiment.

Prior to the entry of a human 300 into the private toilet room 200, in other words, while a human 300 is absent in the private toilet room 200, the UV irradiation unit 10B continuously irradiates the inside of the private toilet 200 with the UV light.

When a human 300 enters the private toilet room 200 from this state, the human detecting sensor 11 detects the presence of the human 300 at the time point P, and the UV irradiation unit 10B stops the irradiation of the UV light into the private toilet room 200.

Thereafter, when the human 300 is seated on the toilet seat 212 in the private toilet room 200, at the time point Q, the pressure sensor 12 detects the seating of the human 300, the timer 1 starts counting, the UV irradiation unit 10B starts the irradiation of the UV light to the inside of the private toilet room 200. Subsequently, after a predetermined time T1 has elapsed from the time point Q, the UV irradiation unit 10B stops the irradiation of the UV light to the inside of the private toilet room 200 and the human 300.

In this way, when a person 300 enters the private toilet room 200, the UV irradiation unit 10B once stops the irradiation of the UV light. Subsequently, when the person 300 is seated on the toilet seat 212, the UV irradiation unit 10B emits the UV light into the private toilet room 200 for a predetermined time T1, and the person 300 in the private toilet room 200 is irradiated with the emitted UV light.

Thereafter, when the person 300 exits from the private toilet room 200, at the time point R, the human detecting sensor 11 detects that the person 300 has exited therefrom, and the controller unit 20 resumes the irradiation of the UV light into the private toilet room 200.

As described above, according to the present embodiment, the controller unit 20 controls the UV irradiation unit 10 to irradiate the space including a human with the UV light for a predetermined time (T1) after detecting that a human is seated on the toilet seat 212 based on the detection signal from the pressure sensor 12 within a period of time when the human is determined to be present in the private toilet room 200 based on the detection signal from the human detecting sensor 11.

In this manner, by irradiating a human present at a predetermined position in the enclosed space with the light including the UV light, it makes it possible to effectively irradiate an intended part of the surface of the human body with the UV light. In addition, the movement of a human sitting on the toilet seat 212 in the private toilet room 200 is relatively small. Therefore, by irradiating a human in the state of sitting on the toilet seat 212 with the UV light, it makes it possible to effectively inactivate harmful microorganisms and/or viruses existing on the surface of the human body (e.g., the surface of skin and clothes).

When the human detecting sensor 11 detects that a human has entered the private toilet room 200, the controller unit 20 controls the UV irradiation unit 10B to once stop the UV light irradiation, and then controls the UV irradiation unit 10B to irradiate the space including the human for a predetermined time (T1) after the pressure sensor 12 detects that the human has been seated on the toilet seat 212.

Therefore, in the case that the private toilet room 200 has been irradiated with the UV light before a human enters the private toilet room 200, it makes it possible to temporarily stop the UV light irradiation when a human enters the private toilet room 200, and then irradiate the human in a state being seated on the toilet seat 212 with the UV light for a predetermined time (T1). As a result, it makes it possible to appropriately perform the UV light irradiation to the inside of the private toilet room 200 in which no human is present and also the UV light irradiation to a human who has entered the private toilet room 200.

Furthermore, the UV irradiation unit 10B is installed on the wall portion 202 of the private toilet room 200 on the assumption that the UV irradiation unit 10B irradiates a human 300 concerned with the UV light when the human 300 takes a posture of sitting on the toilet bowl 211. For this reason, by using the UV irradiation unit 10B to perform the UV light irradiation, it makes it possible to increase the dose amount on the floor surface as compared to the case of using the UV irradiation unit 10A. In other words, it makes it possible to inactivate harmful microorganisms and/or viruses attaching to the floor surface more effectively.

Yet furthermore, the UV irradiation unit 10B is disposed at a position where the UV light is emitted from the back of the head side of a human 300 concerned when the human 300 is in a posture of sitting on the toilet bowl 211. Therefore, it makes it possible to prevent the UV light emitted from the UV irradiation units 10B from directly irradiating the eyes of the human 300. As a result, it makes it possible to suppress the occurrence of eye disorders such as ocular pain, hyperemia, inflammation of the cornea, and the like.

Although the present embodiment describes a certain case in which the UV light irradiation is performed using the UV irradiation unit 10B, it is also possible to use the UV irradiation unit 10A provided on the ceiling 201 of the private toilet room 200.

When the UV irradiation unit 10A is used, the irradiance on the UV irradiated surface of a human 300 sitting on the toilet seat 212 is smaller than the irradiance on the UV irradiated surface of a human 300 standing on the floor, the former is, for example, 0.010 (mW/cm$^2$).

For this reason, when the UV light source provided in the UV irradiation unit 10A is a low pressure mercury lamp, assuming that the number of times N the private toilet room belonging to the hospital room is used per day (i.e., the number of times a patient enters the private toilet room per day) is set to 10 times, the UV light irradiation time (i.e., predetermined time T1) during one stay in the private toilet room is calculated to be 60 seconds according to the above described formula (2).

Alternatively, when the UV light source provided in the UV irradiation unit 10A is a KrCl excimer lamp, assuming that the number of times N the private toilet room belonging to the hospital room is used per day (i.e., the number of times a patient enters the private toilet room per day) is set to 10 times, the UV light irradiation time (i.e., predetermined time T1) during one stay in the private toilet room is calculated to be 220 seconds according to the above described formula (2).

As described above, when the UV irradiation unit 10A is used, it makes it possible to lengthen the UV light irradiation time (i.e., predetermined time T1) as compared to the case in which the UV irradiation unit 10B is used.

In the present embodiment, a certain case has been described in which the pressure sensor 12 disposed on a toilet seat 212 is used as a sensor for detecting a state in which a human is seated on the toilet seat 212 in a private toilet room 200. However, the present embodiment is not limited thereto, and any sensor can be used as long as the state in which a human is seated on the toilet seat 212 can be detected.

Modification 1 to Second Embodiment

In the second embodiment described above, a certain case has been described in which the UV irradiation unit 10A continuously performs the UV light irradiation to the inside of the private toilet room 200 when a human 300 is absent in the private toilet room 200. However, alternatively, the UV light irradiation to the inside of the private toilet room 200 may be performed for a predetermined time T2 when a human is absent in the private toilet room 200.

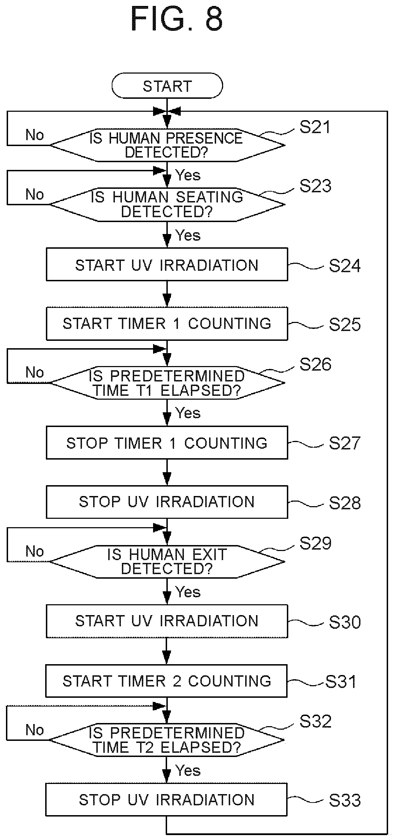

FIG. 8 is a flowchart illustrating an exemplary processing procedure performed by the inactivation apparatus 100 according to the present modification. Referring to FIG. 8, the same step numbers are assigned to the portions that perform the same processing as in FIG. 6, and the following description will focus on the portions that differ in processing.

In step S21, when the controller unit 20 detects the presence of a human 300 in the private toilet room 200, the processing proceeds to step S23.

After the controller unit 20 controls the UV irradiation unit 10B to start emitting the UV light in step S30, the processing proceeds to step S31 and the controller unit 20 starts counting the timer 2, which is another counter.

Subsequently, in step S31, the controller unit 20 determines, based on the count value of the timer 2, whether or not the predetermined time T2 has elapsed since the timer 2 starts counting, in other words, whether or not the predetermined time T2 has elapsed since a human 300 exited from the private toilet room 200. When the predetermined time T2 has not yet elapsed, the controller unit 20 waits until the predetermined time T2 has elapsed. On the other hand, when the controller unit 20 determines that the predetermined time T2 has elapsed, the processing proceeds to step S32.

Here, the predetermined time T2 is set to a time sufficient to inactivate at least a part of harmful microorganisms and/or viruses existing in the private toilet room 200 from which the human 300 has left.

In step S33, the controller unit 20 controls the UV irradiation unit 10B to stop emitting the UV light and the processing returns to step S21.

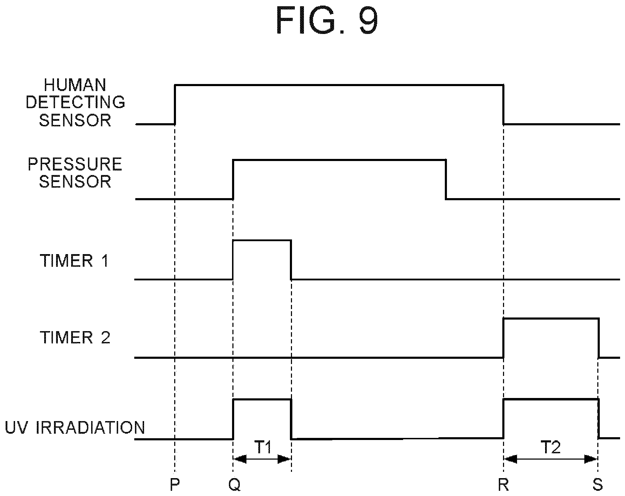

FIG. 9 is a time chart illustrating an exemplary operational sequence performed by the inactivation apparatus 100 according to the present modification.

Here, it is assumed that the UV irradiation unit 10B stops the UV light irradiation to the inside of the private toilet room 200 before a human 300 enters the private toilet room 200.

When a human 300 enters the private toilet room 200 in this state, at the time point P, the human detecting sensor 11 detects the presence of the human 300. Thereafter, when the human 300 is seated on the toilet seat 212, at the time point Q, the pressure sensor 12 detects the seating of the human 300, the timer 1 starts counting, and the controller unit 20 starts the irradiation of the UV light to the inside of the private toilet room 200 and the human 300 therein. Subsequently, after the predetermined time T1 elapses from the time point Q, the controller unit 20 stops the irradiation of the UV light to the inside of the private toilet room 200 and the human 300 therein.

As described above, even when the UV irradiation unit 10A stops the UV light irradiation prior to the entry of a human 300 into the private toilet room 200, the UV irradiation unit 10B starts the UV light irradiation once a human 300 enters the private toilet room 200 and is seated on the toilet seat 212. Thereafter, the UV irradiation unit 10B continues irradiating the human 300 in the private toilet room 200 with the UV light for a period of time from that time point until the predetermined time T1 elapses.

Subsequently, when the human 300 exits from the private toilet room 200, at the time point R, the human detecting sensor 11 detects that the human 300 has exited therefrom, the timer 2 starts counting, and the controller unit resumes the irradiation of the UV light to the inside of the private toilet room 200.

Yet subsequently, when the predetermined time T2 elapses from the time point R at which the human 300 exits from the private toilet room 200, at the time point S, the controller unit 20 stops the irradiation of the UV light to the inside of the private toilet room 200.

As described above, when the controller unit 20 determines via the human detecting sensor 11 that no human is present in the private toilet room 200, the UV irradiation unit 10B may irradiate the inside of the private toilet 200 in which a human is absent with the UV light, and after the UV light irradiation is performed for a certain period of time (i.e., the predetermined time T2), the UV irradiation unit 10B may stop the UV light irradiation. By ensuring that the UV light irradiation is performed only for a certain period of time inside the private toilet room 200 in which a human is absent, it makes it possible to provide a pause time for the UV light source provided in the UV irradiation unit 10B, thereby extending the usable life of the UV light source.

Modification 2 to Second Embodiment

In the second embodiment described above, a certain case has been described in which the human detecting sensor 11 is used to detect the presence of a human 300 in a private toilet room 200. However, the present embodiment is not limited thereto, and alternatively the door sensor 13 may be used to detect the entry and exit of a human 300 into the private toilet room 200.

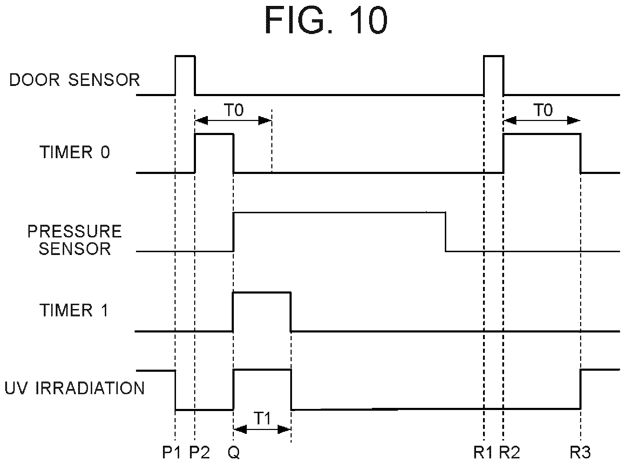

FIG. 10 is a time chart illustrating another exemplary operational sequence performed by the inactivation apparatus 100 according to the present modification.

Prior to the entry of a human 300 into the private toilet room 200, in other words, while a human 300 is absent in the private toilet room 200, the UV irradiation unit 10B continuously irradiates the inside of the private toilet room 200 with the UV light.

When a human 300 opens the door 203 to enter the private toilet room 200 from this state, at the time point P1, the door sensor 13 detects that the door 203 has been opened, and the controller unit 20 stops the irradiation of the UV light to the inside of the private toilet room 200. Subsequently, at the time point P2 when the human 300 enters the private toilet room 200 and the door 203 is closed, the door sensor 13 detects that the door 203 is closed.

Thereafter, at the time point P2, the timer 0 starts counting. The timer 0 is set to terminate counting when a predetermined time T0 has elapsed from the start of the operation, to transmit a counting end signal to the controller unit 20, and to be reset. It should be noted that the timer 0 is set to be reset by the controller unit 20 when the pressure sensor 12 detects that a human 300 is seated on the toilet seat 212 even in the middle of counting.

Here, the predetermined time T0 above described is set to be sufficiently longer than the time from the entry of a human 300 into the private toilet room 200 until the seating on the toilet seat 212.

Subsequently, when the human 300 in the private toilet room 200 is seated on the toilet seat 212, at the time point Q, the pressure sensor 12 detects the seating of the human 300, the timer 1 starts counting and the controller unit 20 starts the irradiation of the UV light to the inside of the private toilet room 200. At this time, the timer 0 terminates counting. Then, after the predetermined time T1 has elapsed from the time point Q, the controller unit 20 stops the irradiation of the UV light to the inside of the private toilet room 200 and the human 300 therein.

As described above, when a human 300 enters the private toilet room 200, the UV irradiation unit 10B once stops the UV light irradiation. Thereafter, when the human 300 is seated on the toilet seat 212, the UV irradiation unit 10B emits the UV light into the private toilet room 200 for the predetermined time T1, and the human 300 in the private toilet room 200 is irradiated with the UV light.

Subsequently, when the human 300 opens the door 203 to exit from the private toilet room 200, at the time point R1, the door sensor 13 detects that the door 203 has been opened. Then, at the time point

R2 when the human 300 exits from the private toilet room 200 and the door 203 is closed, the door sensor 13 detects that the door 203 is closed.

Then, at this time point R2, the timer 0 starts counting. As the human 300 has already exited from the private toilet room 200, even if the predetermined time T0 has elapsed from the time point R2, the pressure sensor 12 does not detect seating on the toilet seat 212. For this reason, at the time point R3 when the predetermined time T0 has elapsed from the time point R2, the controller unit 20 resumes the irradiation of the UV light to the inside of the private toilet room 200.

As described above, even when the door sensor 13 is used instead of the human detecting sensor 11, it makes it possible to achieve the same effect as that of the second embodiment described above by using the counting of the timer 0. In addition, since the door sensor 13 can detect the opening and closing of the door 203, it makes it possible to emit the UV light inside the enclosed space (e.g., private toilet room 200) while the door 203 is closed. As a result, it makes it possible to prevent an object outside the enclosed space from being unintentionally irradiated with the UV light.

Here, a certain case has been described in which the controller unit 20 controls the timer 0 to start counting at the time when the door sensor 13 detects that the door 203 is closed. However, the controller unit 20 may controls the timer 0 to start counting when the seating of a person 300 has not been detected by the pressure sensor 12 at the time when the door sensor 13 detects that the door 203 is closed.

In this case, assuming that the human 300 remains seated on the toilet seat 212 and an undesired opening or closing of the door 203 occurs due to a reason such as forgetting to lock the door 203 or a defect in the door 203, the controller unit 20 can prevent the timer 0 from starting the counting.

In other words, when the controller unit 20 receives the detection signal indicating the seating of a human 300 from the pressure sensor 12, the controller unit 20 may prevent the time 0 from starting the counting unless a detection signal indicating the leaving of the human 300 is next received from the pressure sensor 12, even if the detection signal indicating that the door 203 is closed is received from the door sensor 13.

In this way, by confirming both the detection signal from the door sensor 13 and the detection signal from the pressure sensor 12, it makes it possible to properly determine that no human is present in the private toilet room 200.

Furthermore, as in the modification 1 of the second embodiment described above, when a human is absent in the private toilet room 200, the irradiation of the UV light into the private toilet room 200 may be performed for the predetermined time T2. In other words, the irradiation of the UV light to the inside of the private toilet room 200 may be terminated when the predetermined time T2 has elapsed from the time point R3 in FIG. 10.

Further Modifications to First and Second Embodiments

In the embodiments described above, a certain case has been described in which the inactivation apparatus 100 is installed in a private toilet room. However, the present embodiment is not limited thereto. For example, the inactivation apparatus 100 may be installed in a particularly confined or narrow space in a facility where people frequently gather, such as a hospital room, an elevator, a conference room, or the like.

In addition, the timing of irradiating a human with the UV light from the inactivation apparatus 100 may be any timing during the period in which the human is present in the enclosed space. However, when it is possible to detect a timing during which harmful microorganisms and/or viruses are likely to be dispersed during the period in which a human is present in the enclosed space, it is preferable to irradiate the human with the UV light at such timing.

Also, in the embodiments described above, a certain case has been described in which the inactivation apparatus 100 is installed in an enclosed space in which a human can enter and exit. However, the enclosed space described above may be a space in which an animal other than a human can enter and exit.

Furthermore, in the embodiments described above, UV light is irradiated to a human or a space including a human for the predetermined time T1. However, when the operation of the UV light source is an alternately repetitive operation of light-emitting and non-light-emitting, the sum of the light-emitting operation time may be used as the predetermined time T1.

For example, when the light-emitting operation time of the excimer lamp is set to 10 ms or more and 1000 ms or less by controlling the power supply to the excimer lamp, the subsequent pause time is set to 10 ms or more and 10 seconds or less, and the light-emitting operation and pause are repeated alternately, then the above predetermined time T1 is calculated as the sum of the light-emitting operation time of the repetitive light-emitting operations.

More particularly, for example, when the light-emitting operation time of the KrCl excimer lamp is set to 100 ms, the pause time is set to 100 ms, and the predetermined time T1 is set to 30 seconds, then the number of the light emission operations of KrCl is 300 times, and the operation time of the Kr excimer lamp is 60 seconds including the pause time.

In other words, when the operation of the UV light source is continuous, the UV light irradiation period to a human or a space including a human is equal to the predetermined time T1. On the other hand, when the operation of the UV light source is intermittent, including a pause period, the UV light irradiation period is longer than the predetermined time T1.

For example, when the space including a human is a toilet room, the UV light irradiation period may be set longer by controlling the operation of the UV light source to be intermittent. As a result, it makes it possible to enhance the possibility of performing the UV light irradiation to bacteria and/or viruses dispersed due to defecation or splashing.

It should be noted that, when the light emitting operation time is 10 ms or more and 1000 ms or less and the pause time is 10 ms or more and 10 seconds or less as described above, it is necessary to control the opening and closing of the shutter for light shielding as described above when a low pressure mercury lamp is used. However, in some cases, the opening and closing operation of the shutter is required to be made faster, which is difficult to deal with.

For this reason, as the UV light source, it is preferable to use a light source capable of alternately repeating the UV light emitting operation and the pause time by the power supply control.

For example, excimer lamps (KeCl excimer lamps) and solid state light sources (e.g., light emitting diodes (LEDs), laser diodes (LDs)) as described above can be used as such light sources.

Third Embodiment

Hereinafter, a third embodiment according to the present invention will be described in detail.

In the above-described embodiments, when the presence of a human 300 in the enclosed space (e.g., private toilet room) 200 is detected by the sensor and the UV light is irradiated for a predetermined time T1, the light-emitting operation and the subsequent pause are alternately repeated, and the sum of the light-emitting operation time may be set as the above-described predetermined time T1. In other words, in the above-described embodiments, a certain case has been described in which the presence of a human 300 is detected and then so-called intermittent lighting may be performed where the light-emitting operation and the non-light-emitting operation by the UV irradiation unit are alternately repeated.

The present third embodiment will describe a certain case in which the intermittent lighting is performed without depending on the detection signal from the sensor.

The present embodiment will describe a case in which the UV light irradiation is performed not only in an enclosed space but also in other facilities (e.g., offices, commercial facilities, medical facilities, schools, and the like) or vehicles (e.g., automobiles, trains, buses, airplanes, ships, and the like) in which humans or animals are present, regardless of the presence or absence of a human 300.

The inactivation system according to the present embodiment irradiates a surface or a space in a facility or a vehicle in which a human or an animal is present with UV light, and inactivates microorganisms and/or viruses, which are harmful to a human body or an animal, that exist at least on the surface or in the space in the facility or the vehicle. It should be noted that the term "space in a facility or a vehicle in which a human or an animal is present", which is a target space to be irradiated with the ultraviolet light, is not limited to a space where a human or an animal is actually present but includes a space where a human or an animal may enter and leave but no human or animal actually exists.

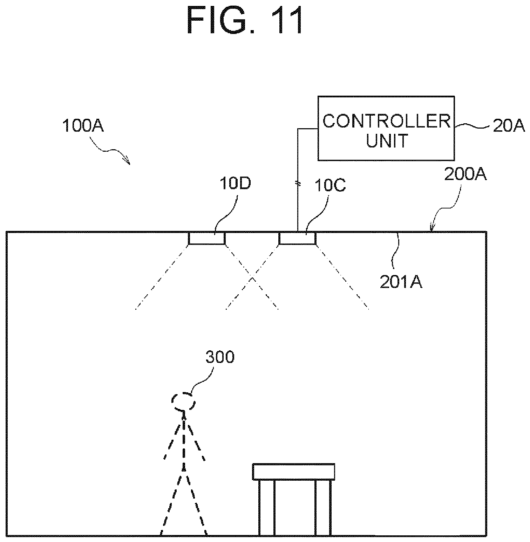

FIG. 11 is a schematic diagram illustrating an exemplary configuration of an inactivation system according to the present embodiment. The inactivation system is provided with an inactivation apparatus 100A. The inactivation apparatus 100A includes an ultraviolet light irradiation unit (i.e., UV irradiation unit) 10C that emits the UV light to surfaces or spaces in a facility 200A, a light source for illumination 10D, and a controller unit 20A.

The UV irradiation unit 10C is installed, for example, on the ceiling 201A in the facility 200A. Any UV irradiation unit 10C is deployable as long as it can emit the UV light to surfaces and spaces within the facility 200A, and the installation position is not particularly limited. The light emitted by the UV irradiation unit 10C includes UV light having the wavelength range of 190 nm to 235 nm, which have less adverse effects on the human bodies.

The UV irradiation unit 10C is equipped with, for example, a KrCl excimer lamp that emits UV light having a center wavelength of 222 nm as a UV light source. The UV irradiation unit 10C may include a wavelength selective filter that transmits only light in the wavelength range of 190 nm to 235 nm and cuts light in other wavelength ranges.

The light source for illumination 10D is installed on the ceiling 201A in the facility 200A. The light source for illumination 10D is assumed to have, for example, an emission spectrum that overlaps at least a part of the wavelength range of 300 nm to 500 nm.

The controller unit 20A controls the irradiation and non-irradiation of light by the UV irradiation unit 10C. More particularly, the controller unit 20A controls the UV irradiation unit 10C to perform the intermittent lighting under a condition corresponding to the wavelength of the UV light emitted by the UV irradiation unit 10C. Here, at least a part of the period during which the intermittent lighting operation of the UV irradiation unit 10C is performed is included in the period during which the light source for illumination 10D is lit (i.e., turned on).

According to the current safety standards, the maximum allowable UV light exposure amount of 222 nm wavelength for one day (i.e., 8 hours) is $D_{max}$ = 22 (mJ/cm$^2$). Therefore, the condition for intermittent lighting should be set such that the integrated light intensity (i.e., total irradiation amount) for 8 hours is within 22 (mJ/cm$^2$).

In other words, when the irradiance on the UV irradiated surface of the human body is W (mW/cm$^2$) and the number of times the light emitting operation is performed per day (i.e., 8 hours) is N, the single light-emitting operation time Ta (sec) of the UV irradiation unit 10C is expressed as follows:

$$Ta \leq D_{max}/(W*N) \qquad\qquad formula\ (3)$$

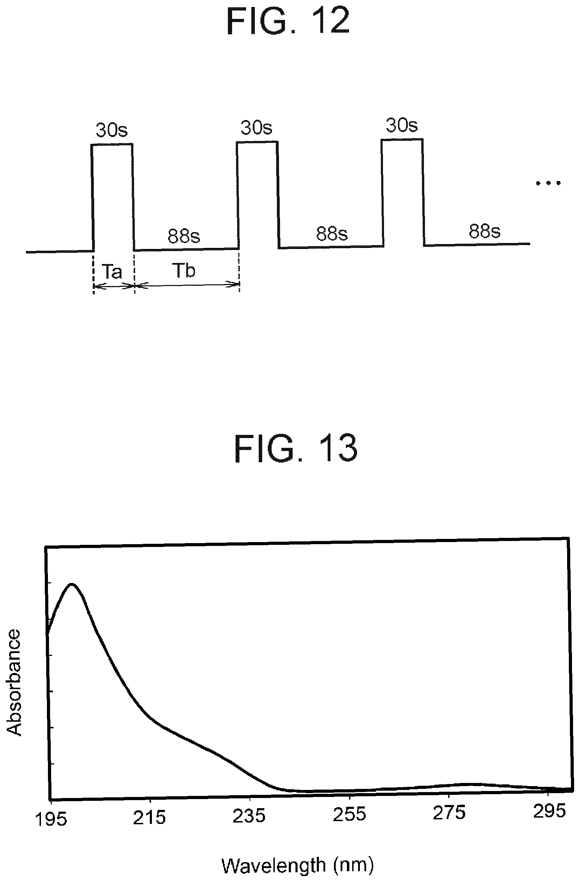

FIG. 12 is a time chart illustrating an exemplary intermittent lighting operation according to the present embodiment.

Referring to FIG. 8, Ta is the light-emitting operation time for one light-emitting operation, and Tb is the non-light-emitting operation time for one non-light-emitting operation. According to the present embodiment, the controller unit 20A switches the light-emitting operation and the non-light-emitting operation of the UV light by controlling the power supply. Hereinafter, the light-emitting operation time Ta is also referred to as the lighting time Ta and the non-light-emitting operation time Tb is also referred to as the pause time Tb in the following explanation.

As shown in FIG. 12, according to the present embodiment, the inactivation apparatus may repeat, for example, the lighting operation of 30 (sec) and the pause operation of 88 (sec). When the above-described operation is repeated for 8 hours, 244 lighting operations will be performed in 8 hours. In other words, the sum of total lighting time in 8 hours amounts to 7348 (sec).

Under those conditions, for example, when the intermittent lighting is performed with the irradiance of 0.0029 $mW/cm^2$, it is possible to keep the amount of irradiation for 8 hours to be within 22 $mJ/cm^2$.

Since there is a pause time during the intermittent lighting, the UV light irradiation period until the irradiation amount (i.e., integrated light intensity) reaches 22 $mJ/cm^2$ is longer than that in continuous lighting, assuming that the UV irradiance is the same. For this reason, it makes it possible to enhance the possibility that UV light irradiation can be carried out against the scattering of bacteria, viruses, and the like caused by the entry and exit of humans or animals into a facility or vehicle so as to increase the effectiveness of inactivation. It should be noted that once the numerical value of the maximum allowable UV light exposure amount $D_{max}$ according to the safety standard is changed, the conditions for intermittent lighting shall be set based on the changed value.

In addition, intermittent lighting makes it possible to extend the usable life of the UV light source (i.e., extend the time until the UV light source needs to be replaced).

Furthermore, the present inventors have newly found that, in the inactivation of microorganisms and viruses using UV light having a wavelength of 200 nm to 230 nm, and in particular, UV light having a wavelength of 222 nm, the same inactivation effect is obtainable regardless of the lighting operation being the continuous lighting or intermittent lighting, as long as the same amount of UV irradiation is applied. The present inventors have also found that, even if the pause time of intermittent lighting is set to be longer, the inactivation effect does not deteriorate. This is because not only the inactivation of microorganisms and/or viruses, but also the proliferation of bacteria during the pause time can be effectively inhibited. Hereinafter, this point will be described in detail.

Bacterial cells contain nucleic acids (DNA and RNA) that carry genetic information. When irradiated with UV light, the nucleic acids absorb the UV light and the DNA bindings are damaged. When irradiated with the UV light, the nucleic acids absorb the light and the DNA bindings are damaged. This causes the transcriptional control of the genes to stagnate, interfering with metabolism and leading to death of bacteria. In other words, UV light does not immediately kill the bacteria themselves, but it does cause them to lose their metabolic and proliferative capabilities. For this reason, the expression "inactivation" is generally used.

However, some bacteria cause DNA damage to be repaired when being irradiated with light having a wavelength of 300 nm to 500 nm after the DNA has been damaged by UV light irradiation at a wavelength of 254 nm. This is due to the action of photo-reactivating enzymes (e.g., FAD (flavin adenine dinucleotide)) possessed by bacteria, and this phenomenon is called "photoreactivation of bacteria". The wavelength range of 300 nm to 500 nm also includes visible light from sunlight or white illumination, and it is known that bacterial photoreactivation progresses in a bright environment.

On the other hand, when bacteria are inactivated by UV light having a wavelength of 200 nm to 230 nm, and in particular, by UV light having a wavelength of 222 nm, the photoreactivation of bacteria does not occur even when the bacteria are irradiated with the above-mentioned visible light after being irradiated with the UV light. In other words, the above-described "photoreactivation of bacteria" is inhibited by UV light irradiation of 222 nm wavelength.

FAD, which is a photo-reactivating enzyme, includes riboflavin, which acts on photoreactivation, and adenine nucleotide (ADP), which is further classified into adenosine and phosphate.

The absorbance of FAD is similar between UV light having a wavelength of 222 nm and UV light having a wavelength of 254 nm. On the other hand, the absorbance of riboflavin, which acts on the photoreactivation, is greater for UV light having a wavelength of 215 nm to 230 nm than for UV light having a wavelength of 254 nm. This suggests that UV light having a wavelength of 215 nm to 230 nm acts more effectively on riboflavin, thereby inhibiting its function of the photoreactivation. Furthermore, the peak absorbance value of riboflavin in the wavelength range of 200 nm to 230 nm lies near 222 nm, suggesting that UV light irradiation at the wavelength of 222 nm could significantly inhibit the "photoreactivation of bacteria".

In addition, the absorbance of adenosine is greater for UV light having a wavelength of 254 nm than for UV light in the wavelength range of 218 nm to 245 nm. In other words, it can be inferred that UV light having a wavelength of 254 nm is easily absorbed by adenosine, or in other words, adenosine acts as a protective barrier against the UV light having a wavelength of 254 nm, making it difficult to act effectively on riboflavin. Therefore, UV light in the wavelength range of 218 nm to 245 nm are more likely to act effectively on riboflavin. In view of the above, UV light having a wavelength of 222 nm is a light that satisfies any of the above effective ranges and can effectively inhibit the photoreactivation effect of bacteria.

Furthermore, the present inventors have further verified the inhibitory effect of UV light having a center wavelength shorter than 222 nm on "photoreactivation of bacteria", and found that even UV light having a center wavelength of 207 nm inhibits the photoreactivation of bacteria. It should be noted that the absorbance of riboflavin is lower in the UV light having a wavelength of 207 nm than in the UV light having a wavelength of 254 nm. In other words, it has been turned out that the photoreactivation of bacteria is effectively inhibited even in the wavelength range where the absorbance of riboflavin is lower than that of UV light having the wavelength of 254 nm. It can be inferred that the photoreactivation of bacteria is inhibited as a result of the effective action of the UV light having the short wavelength band on the cellular tissues that constitute microorganisms or viruses such as bacteria and fungi.

FIG. 13 is a chart illustrating the characteristics of the absorption wavelength of the protein. It can be observed that the absorption rate for proteins increases in the wavelength band shorter than 240 nm. Therefore, the UV light having the wavelength shorter than 240 nm is effectively absorbed by proteins, which are components of the cell membranes or enzymes of bacteria and viruses. In particular, bacteria and viruses are physically much smaller than human cells, and the UV light transmits therethrough easily even in the wavelength range shorter than 240 nm. In other words, it is presumed that the UV light having the wavelength band shorter than 240 nm (e.g., 190 nm to 235 nm) is capable of inactivating microorganisms and/or viruses while suppressing their adverse effects on humans or animals, and furthermore, by effectively acting on the cellular tissues that constitute bacteria and viruses, it makes it possible to enhance the effect of suppressing bacterial functions such as photoreactivation. As a result, it is considered that the UV light having the wavelength of 190 nm to 235 nm is capable of efficiently performing inactivation even with the intermittent UV light irradiation, because the inactivation proceeds while suppressing the photoreactivation of bacteria from occurring.

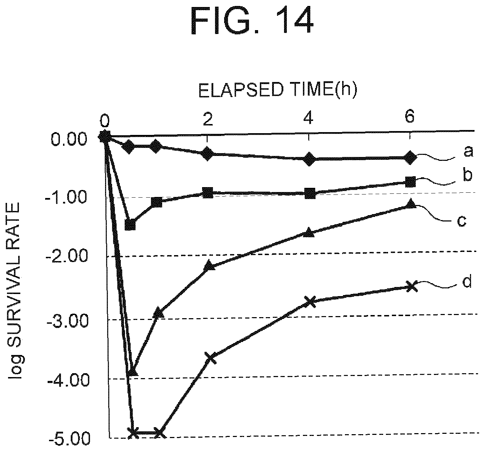

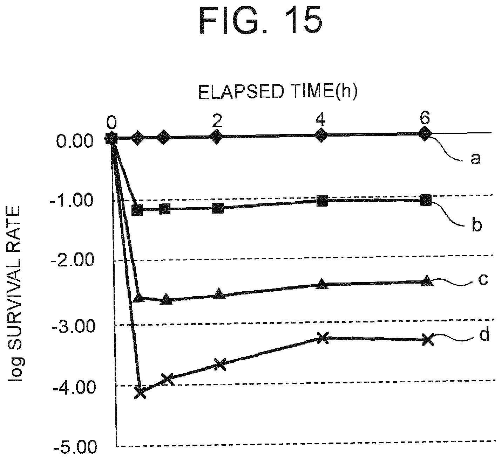

FIG. 14 is a chart exemplarily illustrates the results of the photoreactivation experiment of bacteria irradiated with UV light having a wavelength of 254 nm, and FIG. 15 is a chart exemplarily illustrates the results of the photoreactivation experiment of bacteria irradiated with UV light having a wavelength of 222 nm. Here, the bacterium to be inactivated was Staphylococcus aureus, which is easily sterilized by the UV light having a wavelength of 254 nm, and UV light irradiation was performed in an environment where visible light including light having a wavelength of 300 nm to 500 nm was irradiated, and the change in the survival rate of the bacteria after UV irradiation was confirmed.

Referring to FIGs. 14 and 15, the horizontal axis denotes the elapsed time (h) and the vertical axis denotes the log survival rate of the bacteria. In FIGs. 14 and 15, the experimental results a to d show the change in the survival rate of bacteria when the UV irradiation amount is 0 mJ/cm$^2$, 5 mJ/cm$^2$, 10 mJ/cm$^2$, and 15 mJ/cm$^2$, respectively.

As shown in FIG. 14, the survival rate of the bacteria increases over time. In other words, the bacteria are photo-reactivated after UV light irradiation at a wavelength of 254 nm in an environment where visible light is irradiated. More particularly, the number of surviving bacteria recovers significantly in about one to two hours after visible light irradiation.

In contrast, as shown in FIG. 15, when the UV light of 222 nm wavelength is irradiated, no photoreactivation of the bacteria is observed even when visible light is irradiated. In other words, the photoreactivation of the bacteria is inhibited.

Bacteria whose photoreactivation is inhibited will not proliferate and will be inactivated, as their DNA will remain damaged. For this reason, the UV light irradiation at a wavelength of 222 nm can effectively reduce the recovery and proliferation of bacteria.

As a result, an inactivation system that irradiates bacteria with UV light having a wavelength of 222 nm are particularly effective in environments where bacteria can be easily photo-reactivated, in particular, in environments where visible light including light having a wavelength of 300 nm to 500 nm is irradiated.

In the inactivation system using UV light irradiation at a wavelength of 254 nm, microorganisms or viruses that are not photo-reactivated (e.g., Bacillus subtilis (so-called Bacillus subtilis natto), influenza, etc.) can be effectively inactivated. On the other hand, bacteria that are photo-reactivated (e.g., Escherichia coli, Salmonella, etc.) are difficult to inactivate in an environment where visible light is irradiated. For this reason, such inactivation system is likely to create an environment in which only certain bacteria having photo-reactivating enzymes are easy to survive, and there is concern that this may increase the risk of infection by such bacteria.

For example, when Bacillus subtilis, which is harmless, coexists with Escherichia coli, which is harmful, the antibacterial substances produced by Bacillus subtilis can kill Escherichia coli. However, when Bacillus subtilis and Escherichia coli are inactivated by UV light irradiation at a wavelength of 254 nm, a situation is created in which Bacillus subtilis cannot be revived but Escherichia coli can be revived. In this case, there is a concern that the risk of infection by Escherichia coli may be increased.

On the other hand, if the photoreactivation of harmful bacteria can be inhibited by irradiation with UV light having a wavelength of 200 nm to 230 nm, in particular, UV light having a wavelength of 222 nm, the risk of infection by such bacteria can be reduced.

In addition, if the photoreactivation of the bacteria can be inhibited, it makes it possible to suppress the viruses from proliferating through the bacteria.

For example, viruses that infect bacteria (i.e., bacteriophages) are known to proliferate through bacteria as a vector. Viruses may proliferate by infecting bacteria as a bacterial vector. This bacteriophage is a generic term for viruses that infect bacteria but may be harmful to humans. For example, lysogenic phages occasionally have toxic or drug-resistance genes in their genomes, and it has been pointed out that these may cause harm to humans indirectly through bacteria. Examples are the toxins of cholera and diphtheria.

For this reason, inhibiting the photoreactivation of bacteria will also prevent the viruses such as phages from proliferating.

As described above, by irradiating object surfaces and spaces inside facilities or vehicles where humans or animals are present with UV light having a wavelength of 200 nm to 230 nm, and in particular with UV light having a wavelength of 222 nm, it makes it possible to inactivate harmful microorganisms and/or viruses inside facilities or vehicles, and also to effectively suppress the photoreactivation of bacteria after UV irradiation. As a result, it makes it possible to prevent viruses such as bacteriophages from proliferating.

Furthermore, UV irradiation at a wavelength of 200 nm to 230 nm, in particular 222 nm, can inhibit the photoreactivation of bacteria, so that the inactivation effect is kept maintained even during a pause time (i.e., rest time) in which no UV light is irradiated. In other words, the inactivation effect of the intermittent lighting is equivalent to that of the continuous lighting.

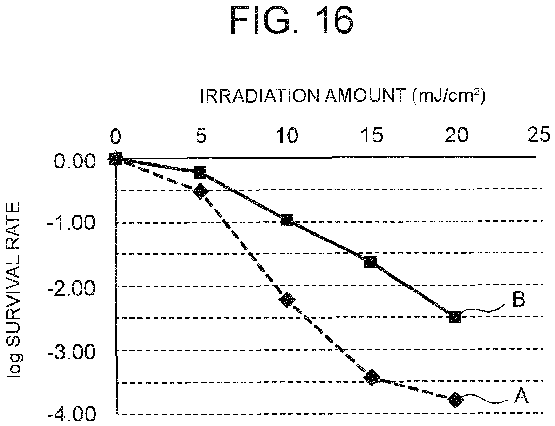

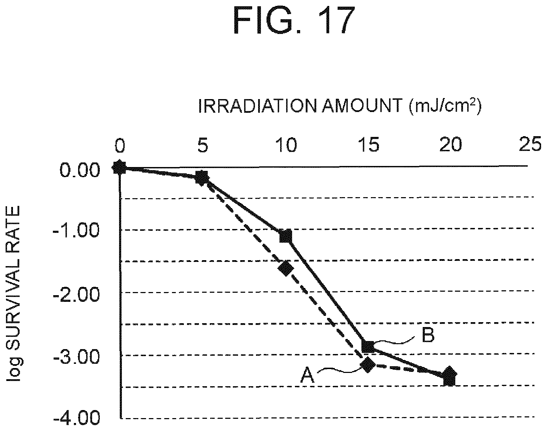

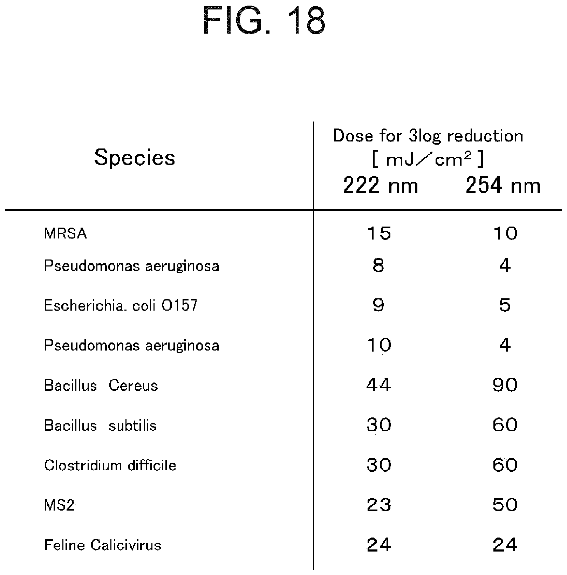

FIG. 16 is a chart exemplarily illustrating the results of comparison between the continuous lighting and the intermittent lighting using UV light having a wavelength of 254 nm, and FIG. 17 is a chart exemplarily illustrating the results of comparison between the continuous lighting and the intermittent lighting using UV light having a wavelength of 222 nm. Here, the bacterium to be inactivated was Staphylococcus aureus, which is easily sterilized by the UV light having the wavelength of 254 nm as shown in FIG. 18, and the change in the survival rate of the bacteria was confirmed between the case where the above UV light is turned on continuously and the case where the above UV light is turned on intermittently in an environment where the visible light is being irradiated. Referring to FIGs. 16 and 17, the horizontal axis denotes the irradiation amount of UV light (i.e., integrated light intensity) ($mJ/cm^2$) and the vertical axis denotes the log survival rate of the bacteria. In FIGs. 16 and 17, the dashed line A denotes the results of the continuous lighting, and the solid line B denotes the results of the intermittent lighting.

The UV irradiance in the continuous lighting was set to 0.1 ($mW/cm^2$).

The conditions for the intermittent lighting were set to lighting time Ta = 50 (sec), pause time Tb = 59 minutes and 10 seconds (i.e., 3,550 (sec)), and lighting duty ratio = 1.39 (%). The lighting duty ratio is the ratio of the lighting time Ta to the total sum of the lighting time Ta and the pause time Tb, and is the value expressed by Td = Ta/(Ta + Tnb). The UV irradiance during lighting was set to 0.1 ($mW/cm^2$), and the irradiation amount of UV light per lighting operation was set to 5 ($mJ/cm^2$).

As shown in FIG. 16, in the case of UV light irradiation at a wavelength of 254 nm, the inactivation effect of the intermittent lighting is inferior to that of the continuous lighting. It is considered to be due to that fact that the bacteria are photo-reactivated during the pause time of the intermittent lighting. Thus, UV light irradiation at a wavelength of 254 nm with the intermittent lighting cannot ensure the inactivation of the bacteria because the bacteria exert a photoreactivation effect in case of the UV light irradiation at a wavelength of 254 nm.

On the other hand, as shown in FIG. 17, in the case of UV light irradiation at a wavelength of 222 nm, the inactivation effect is equivalent between the intermittent lighting and the continuous lighting, because the photoreactivation of bacteria is inhibited.

Furthermore, as shown in FIGs. 16 and 17, when the bacterium to be inactivated is Staphylococcus aureus, the inactivation effect of using UV light at a wavelength of 254 nm is higher than that of using UV light at a wavelength of 222 nm at any UV irradiation amount in the continuous lighting (as shown in FIG.18). In contrast, in the case of the intermittent lighting, the inactivation effect is higher when 222 nm wavelength UV light is used at any UV irradiation amount (irradiance level).

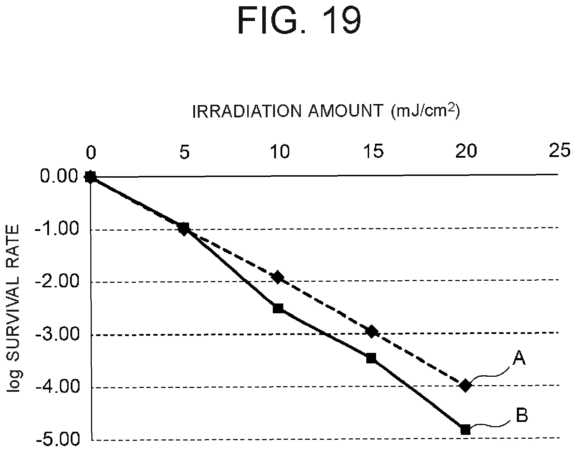

FIG. 19 is a chart exemplarily illustrating the comparison results between the continuous lighting and the intermittent lighting using UV light having a center wavelength of 207 nm. A KrBr excimer lamp is used as the UV light source. Similarly to the comparison results shown in FIGs. 16 and 17, the bacteria to be inactivated was Staphylococcus aureus, and the change in the survival rate of the bacteria was confirmed between the case where the above UV light is turned on continuously and the case where the above UV light is turned on intermittently in the environment where visible light is being irradiated. The horizontal axis denotes the irradiation amount of UV light (i.e., integrated light intensity) ($mJ/cm^2$) and the vertical axis denotes the log survival rate of bacteria. In FIG. 19, the dashed line A denotes the results of the continuous lighting, and the solid line B denotes the results of the intermittent lighting. Also similarly to the comparison results shown in FIGs. 16 and 17, The UV irradiance in the continuous lighting was set to 0.1 ($mW/cm^2$).

The conditions for the intermittent lighting were set to lighting time Ta = 50 (sec), pause time Tb = 59 minutes and 10 seconds (i.e., 3,550 (sec)), and lighting duty ratio = 1.39 (%). The lighting duty ratio is the ratio of the lighting time Ta to the total sum of the lighting time Ta and the pause time Tb, and is the value expressed by Td = Ta/(Ta + Tnb). The UV irradiance during lighting was set to 0.1 ($mW/cm^2$), and the irradiation amount of UV light per lighting operation was set to 5 ($mJ/cm^2$).

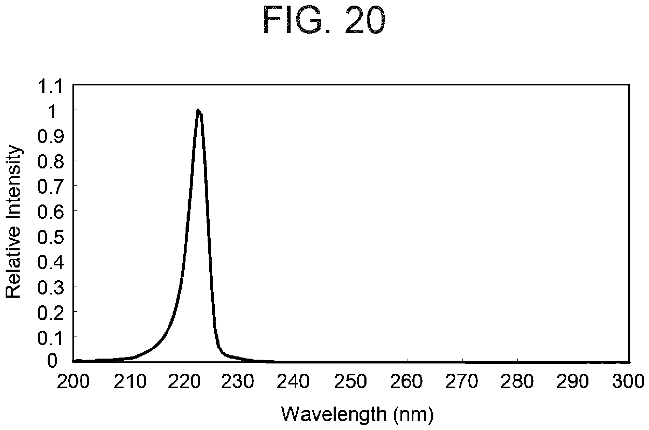

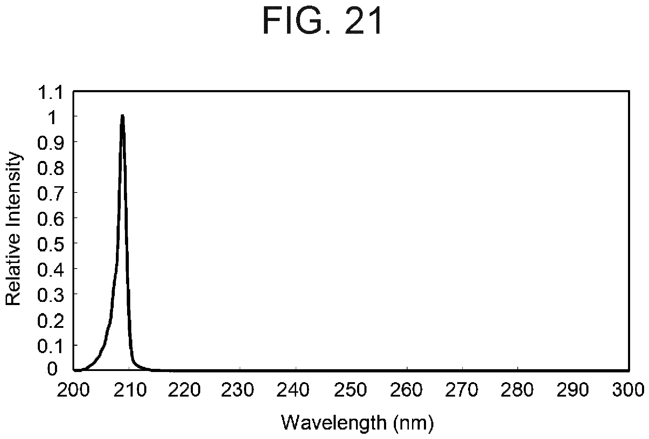

As shown in FIG. 19, even in the case of UV light irradiation at a wavelength of 207 nm, it has been observed that the equivalent inactivation effects are obtainable between the intermittent lighting and the continuous lighting. This is considered to be because the UV irradiation inhibits the photoreactivation of the bacteria itself, which is the similar result to the case of UV light irradiation at a wavelength of 222 nm. FIG. 20 is a chart exemplarily illustrating the emission spectrum of the KrCl excimer lamp with a center wavelength of 222 nm. FIG. 21 is a chart exemplarily illustrating the emission spectrum of the KrBr excimer lamp with a center wavelength of 207 nm. Both UV light sources use wavelength selective filters to limit the emission of UVC light having the wavelength of 240 nm or more, and emit UV light belonging to the wavelength band of 190 to 235 nm.

As shown in FIGs. 20 and 21, even with UV light having different center wavelengths, the equivalent inactivation effects are obtainable between the intermittent lighting and the continuous lighting due to the fact that the photoreactivation of bacteria is inhibited, as shown in FIGs. 17 and 19. This is considered to be due to the fact that the optical absorption rate for proteins is particularly high in the wavelength band shorter than 240 nm, which effectively acts on the cellular tissues constituting bacteria or viruses, especially the cell membrane and enzymes containing protein components.

For this reason, it is presumed that the UV light having the wavelength range shorter than 240 nm (e.g., 190 nm to 235 nm) is capable of inactivating microorganisms and/or viruses while suppressing their adverse effects on humans and animals, and furthermore, by effectively acting on the cellular tissue that constitutes bacteria or viruses, the effect of inhibiting bacterial functions such as photoreactivation is enhanced. In addition, it is presumed that the effect of inhibiting bacterial functions such as photoreactivation is enhanced in the wavelength band of 190 nm to 230 nm more preferably. It makes it possible to effectively inactivate bacteria and/or viruses even in the case of the intermittent irradiation with UV light.

It should be noted that, when the atmosphere is irradiated with UV light having a wavelength less than 190 nm, oxygen molecules existing in the atmosphere are photodegraded to produce more oxygen atoms, and the binding reaction between oxygen molecules and oxygen atoms produces more ozone. For this reason, it is not desirable to irradiate the atmosphere with UV light having the wavelength less than 190 nm. In order to suppress the ozone from being generated more effectively, it is preferable that the UV light to be mainly emitted has a wavelength equal to or greater than 200 nm. As a result, it is preferable to use UV light having a wavelength of 200 nm to 230 nm to inactivate bacteria or viruses existing in the environment.

Meanwhile, in the intermittent lighting using UV light having a wavelength of 222 nm, the inactivation effect does not deteriorate even with a longer pause time.

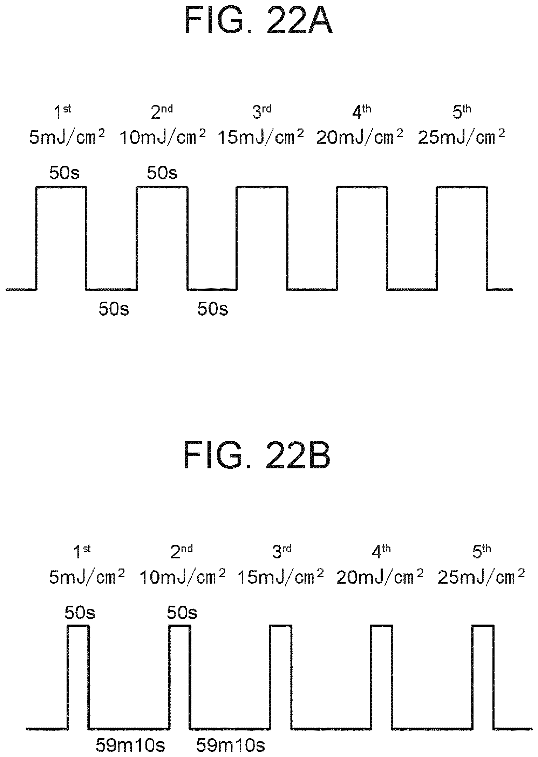

FIG. 22A is a time chart exemplarily illustrating an Operation Example 1 of the intermittent lighting using UV light having a wavelength of 222 nm, and FIG. 22B is a time chart exemplarily illustrating an Operation Example 2 of the intermittent lighting using UV light having a wavelength of 222 nm. The UV irradiance at the time of lighting and the lighting time Ta per lighting (i.e., at one time) are the same between the Operation Example 1 and the Operation Example 2, and only the pause time Tb per pause (i.e., at one time) is different.

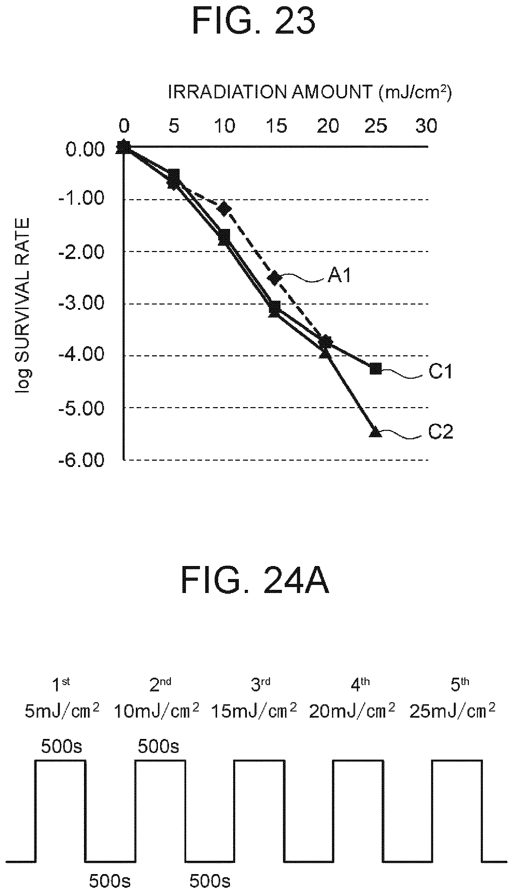

FIG. 23 is a chart exemplarily illustrating the inactivation effect when the intermittent lighting is performed in the Operating Examples 1 and 2. Referring to FIG. 23, the experimental results C1 and C2 denote the change in the survival rate of bacteria when the intermittent lighting is performed in the Operation Examples 1 and 2, respectively. The experimental result A1 denotes the change in the survival rate of bacteria when the continuous lighting is performed with the same UV irradiance during lighting as in the Operation Examples 1 and 2.

Here, as shown in FIG. 22A, the Operation Example 1 has a lighting time Ta = 50 (sec) and a pause time Tb = 50 (sec), that is, the lighting duty ratio is set to 50%. As shown in FIG. 22B, the Operation Example 2 has the lighting time Ta = 50 (sec) and the pause time Tb = 59 minutes and 10 seconds (i.e., 3,550 (sec)), that is, the lighting duty ratio is set to 1.39%.

In both Operation Examples 1 and 2, the UV irradiance at the time of lighting is set to 0.1 (mW/cm$^2$).

In other words, the UV irradiation amount by the first lighting operation (i.e., per one lighting operation) is 5 mJ/cm$^2$ in both cases, and thereafter, the UV irradiation amount through the second, third, ... lighting operations is set to 10 mJ/cm$^2$, 15 mJ/cm$^2$, ..., respectively.

Thus, although the UV irradiation amount by one lighting operation is the same between the intermittent lighting of the Operation Example 1 and that of the Operation Example 2, the pause time of the Operation Example 2 is longer than that of the Operation Example 1. Nevertheless, as shown in FIG. 23, the inactivation effects of the Operation Examples 1 and 2 are substantially the same, and it can be confirmed that the inactivation effect does not deteriorate even if the pause time is set to be longer.

It can also be confirmed that the inactivation effect of the Operation Examples 1 and 2 is substantially the same as the inactivation effect of the continuous lighting at the same UV irradiance.

Also examined was the inactivation effect in the case of low UV irradiance ($\mu$W/cm$^2$) at the time of lighting.

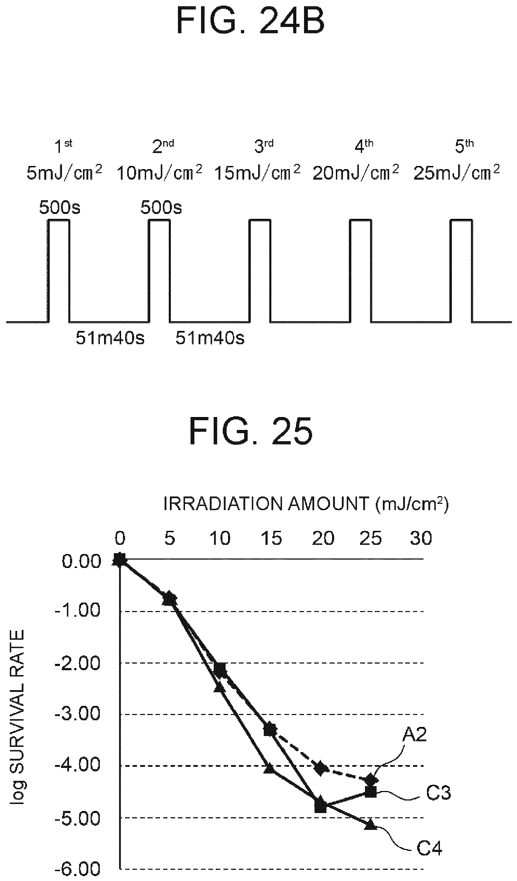

FIG. 24A is a time chart exemplarily illustrating an Operation Example 3 of the intermittent lighting using UV light having a wavelength of 222 nm, and FIG. 24B is a time chart exemplarily illustrating an Operation Example 4 of the intermittent lighting using UV light having a wavelength of 222 nm. The UV irradiance at the time of lighting and the lighting time Ta at one time are the same between the Operation Example 3 and the Operation Example 4, and only the pause time Tb at one time is different therebetween.

FIG. 25 is a chart exemplarily illustrating the inactivation effect when the intermittent lighting is performed in the Operating Examples 3 and 4. Referring to FIG. 25, experimental results C3 and C4 denote the change in the survival rate of bacteria when the intermittent lighting is performed in the Operating Examples 3 and 4, respectively. The experimental result A2 denotes the change in the survival rate of bacteria when the continuous lighting is performed with the same UV irradiance at the time of lighting as in the Operation Examples 3 and 4.

Here, as shown in FIG. 24A, the Operation Example 3 has the lighting time Ta = 500 (sec) and the pause time Tb = 500 (sec), that is, the lighting duty ratio is set to 50%.

As shown in FIG. 15B, the Operation Example 4 has the lighting time Ta = 500 (sec) and the pause time Tb = 51 minutes and 40 seconds (i.e., 3,100 (sec)), that is, the lighting duty ratio is set to 13.9%.

In both of the Operation Examples 3 and 4, the UV irradiance at the time of lighting is set to 0.01 (mW/cm$^2$). In other words, the UV irradiation amount by the first lighting operation (i.e., per one lighting operation) is 5 mJ/cm$^2$ in each case, which is the same as in the Operation Examples 1 and 2 above, and thereafter, the UV irradiation amount through the second, third, ... lighting operations is set to 10 mJ/cm$^2$, 15 mJ/cm$^2$, ..., respectively.

Thus, although the UV irradiation amount by one lighting operation is the same between the intermittent lighting of the Operation Examples 3 and 4 and that of the Operation Examples 1 and 2, the UV irradiance in the Operation Examples 3 and 4 is lower than that of the Operation Examples 1 and 2. Nevertheless, as shown in FIGs. 23 and 25, similarly to the inactivation effect of the Operation Examples 1 and 2, it can be confirmed that the inactivation effects of the Operation Examples 3 and 4 with relatively low UV irradiance at the time of lighting does not deteriorate even if the pause time is set to be longer, and the inactivation effect can be maintained.

It can also be confirmed that the inactivation effect of the Operation Examples 3 and 4 is substantially the same as the inactivation effect of the continuous lighting at the same UV irradiance.

As described above, the inactivation apparatus 100A according to the present embodiment includes the ultraviolet light irradiation unit (i.e., UV irradiation unit) 10C that irradiates object surfaces and spaces in the facility 200A where humans or animals are present with UV light having a wavelength of 222 nm that inactivates microorganisms and/or viruses harmful to the human bodies or animals. The inactivation apparatus 100A also includes the controller unit 20A that controls the irradiation and non-irradiation of UV light by the UV irradiation unit 10C. The controller unit 20A controls the UV irradiation unit 10C to perform the intermittent lighting such that the light-emitting operation (lighting operation) and the non-light-emitting operation (pause operation) by the UV irradiation unit 10C are alternately repeated in accordance with the wavelength of the UV light irradiated from the UV irradiation unit 10C.

More particularly, the controller unit 20A controls the intermittent lighting by the UV light having a wavelength of 222 nm in the facility where humans or animals are present, under the condition that the daily UV irradiation amount (i.e., integrated light intensity) is within the maximum allowable UV exposure amount $D_{max}$ specified by the ACGIH. It makes it possible to inactivate harmful microorganisms and/or viruses present in the facility while appropriately suppressing the adverse effects of UV light on humans and animals.

In addition, since the intermittent lighting is performed, the period until the integrated light intensity of UV light reaches the maximum allowable UV exposure amount $D_{max}$ can be longer than the period until the integrated light intensity of UV light reaches the maximum allowable UV exposure amount $D_{max}$ with continuous lighting at the same UV irradiance. As a result, it makes it possible to enhance the possibility of inactivating harmful microorganisms and/or viruses scattered in the facility, and to lengthen the usable life of the UV irradiation unit 10C (i.e., the time until the UV light source needs to be replaced) as compared to the case of continuous lighting.

In addition, the UV irradiation unit 10C can effectively inhibit the photoreactivation of bacteria by irradiating the target object or space with UV light having a wavelength of 222 nm. Therefore, even in an environment where visible light is irradiated from the light source for illumination 10D, it makes it possible to prevent the bacteria, which have been inactivated during the lighting time, from being photo-reactivated during the pause time without the UV light irradiation so as to maintain the inactivation effect. In other words, the inactivation effect of the intermittent lighting is equivalent to the inactivation effect of the continuous lighting.

Here, the conditions for intermittent lighting can be set according to the integrated light intensity by one lighting operation, the irradiance during lighting operation, the lighting time Ta, the pause time Tb, and the lighting duty ratio Td.

For example, the integrated light intensity per one lighting operation may be equal to or less than 10 mJ/cm$^2$. Conventionally, in an inactivation system, the integrated light intensity of UV light has been set to be more than the equivalent of the amount of energy required for sterilization in order to significantly reduce the bacteria to be sterilized (for example, 99.9% sterilization) with a single UV light irradiation. In addition, taking the problem of the photoreactivation of bacteria into consideration, the need to further increase the integrated light intensity per one time has been conventionally considered when intermittent lighting is used.

FIG. 18 is a chart exemplarily illustrating the results of measuring the amount of energy required to inactivate microorganisms or viruses (i.e., inactivation rate of 99.9%) with UV light having a center wavelength of 222 nm among light in the wavelength range of 190 nm to 235 nm, in which adverse effects on humans and animals are suppressed, and with UV light having a wavelength of 254 nm, which has been conventionally used. Here, the irradiance of the UV light is set at 10 $\mu$W/cm$^2$.

Those results show that the amount of energy required to inactivate bacteria or viruses is somewhat different between UV light of 222 nm and UV light of 254 nm. For example, the integrated light intensity of 15 mJ/cm$^2$ is required for sterilizing methicillin-resistant Staphylococcus aureus (MRSA) with 222 nm UV light.

According to the present embodiment, by intermittent lighting using light in the wavelength range that can inhibit the photoreactivation of bacteria, a higher inactivation effect can be attained even when the integrated light intensity per one time is kept low. More particularly, even when the amount of UV light irradiated per one time is lower than the amount of light that can inactivate the microorganism or virus to be inactivated, the microorganism or virus to be inactivated can be appropriately inactivated by repeating the intermittent lighting. For example, although the irradiation amount of UV light required for 99.9% sterilization of Staphylococcus aureus is about 15 $mJ/cm^2$, even if the intermittent lighting is performed with an integrated light intensity of 5 $mJ/cm^2$ per one lighting operation, the inactivation effect can be appropriately attained, as shown in FIGs. 23 and 25.

Furthermore, the integrated light intensity per one lighting operation may be 5 $mJ/cm^2$ or less.

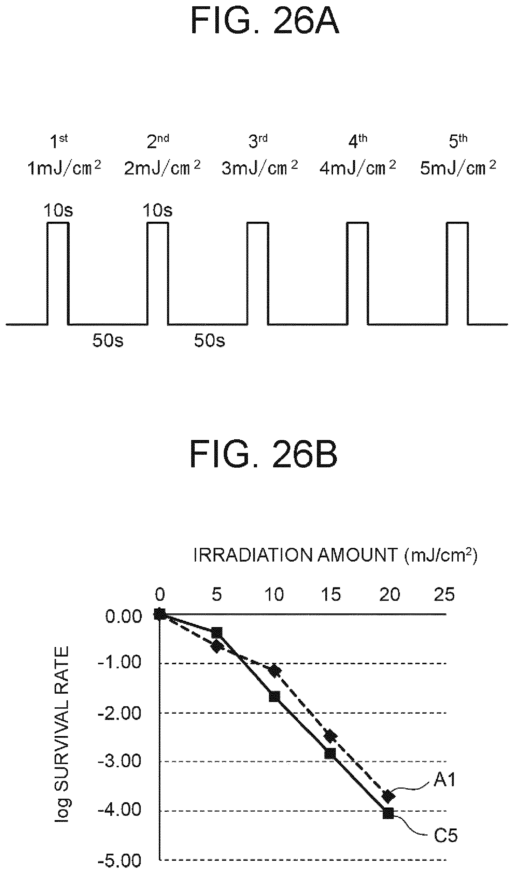

FIG. 26A is a time chart exemplarily illustrating an Operation Example 5, in which the integrated light intensity by one lighting operation is set to 1 $mJ/cm^2$. In the Operation Example 5, the lighting time Ta = 10 (sec), the pause time Tb = 50 (sec), and the UV irradiance during lighting is set to 0.1 ($mW/cm^2$). In other words, the integrated light intensity by the first lighting operation is 1 $mJ/cm^2$, and thereafter, the integrated light intensity through the second, third, ... lighting operations is set to 2 $mJ/cm^2$, 3 $mJ/cm^2$, ..., respectively. The bacterium to be inactivated was Staphylococcus aureus.

FIG. 26B is a chart exemplarily illustrating the inactivation effect when the intermittent lighting is performed in the Operation Example 5. In FIG. 26B, the experimental result C5 denotes the change in the survival rate of bacteria when the intermittent lighting is performed in the Operation Example 5, and the experimental result A1 denotes the change in the survival rate of bacteria when the continuous lighting is performed with the same UV irradiance at the time of lighting as in the Operation Example 5. This experimental result A1 is the same as the experimental result A1 shown in FIG. 23. As shown in FIG. 26B, the inactivation effect can be appropriately attained even when the integrated light intensity by one lighting operation is 1 $mJ/cm^2$, which is less than 5 $mJ/cm^2$.

Also, the integrated light intensity per one lighting operation may be set to a lower value. For example, the integrated light intensity of a single lighting operation may be set to 0.2 $\mu J/cm^2$.

The lighting duty ratio Td may be set to, for example, 50% or less. Also in this case, the inactivation effect can be appropriately attained as shown in FIGs. 23 and 25. In addition, by setting the lighting duty ratio Td to 50% or less, it makes it possible to extend the time for which the inactivated environment can be maintained more than twice with the same integrated light intensity as compared to the case of the continuous lighting.

The lighting duty ratio Td may be set to 25% or less or 10% or less to maintain a more inactivated environment.

Furthermore, the lighting duty ratio Td may be set to be between 1% and 5%, for example. In this case as well, the inactivation effect can be appropriately attained as shown in the experimental results C2 in FIG. 23 and C4 in FIG. 25. In addition, in this case, it makes it possible to further extend the time for which the inactivated environment can be maintained.

Yet furthermore, the lighting time Ta per one time may be equal to or less than one minute. In this case as well, the inactivation effect can be appropriately attained as shown in FIG. 23. According to the present embodiment, since an excimer lamp (e.g., KrCl excimer lamp, KrBr excimer lamp, and the like), which has a shorter rise time of light output than the conventional mercury lamp, is used as the UV light source, even when the lighting time Ta is one minute or less, it makes it possible to attain the stable light output and effectively create the inactivation environment.

According to the present embodiment, it has been described the case in which the excimer lamp is used as the UV light source with shorter rise time of light output, but solid-state light sources (light-emitting diodes (LEDs), laser diodes (LDs), and the like) may also be used.

In the operation cycle of the lighting operation and the pause operation, the pause time Tb of two hours or more may be set for at least a part of the operation cycle. Since the present embodiment can inhibit the photoreactivation of bacteria, the inactivation effect can be appropriately sustained, even when the pause time Tb is set longer than the time required for the photoreactivation of bacteria. It should be noted that, as shown in FIG. 14, when irradiating with UV light having a wavelength of 254 nm, the time required for photoreactivation of bacteria is about 1 to 2 hours.

On the other hand, in the operation cycle of the lighting operation and the pause operation, it is preferable that the pause time Tb of one time for the UV irradiation unit is set to one hour or less. In order to prevent the spread of infection in a facility or a vehicle, it is preferable to shorten the pause time Tb during a period when humans or animals are coming and going.

For example, the airborne infection of viruses is supposed to spread in a state that viruses are attached to minute aerosols of less than 1 $\mu m$ in the air. In this case, the time that the minute aerosol floats in the air is long, and depending on the type of virus, some viruses have a survival time in the aerosol of more than one hour (for example, new coronaviruses).

In order to effectively irradiate such viruses with UV light, it is preferable to control the pause time Tb to be one hour or less. It makes it possible to appropriately inactivate viruses surviving in the aerosol so as to reduce the risk of infection when a human newly enters the facility or the vehicle. In addition, by setting the pause time Tb further shorter, it makes it possible to perform the UV light irradiation multiple times in the aerosol so as to increase the inactivation effect.

In addition, the route of infection through humans and animals needs to be taken into consideration in order to suppress the spread of infection.

When humans sneeze, the droplets can be roughly classified into large particles of 5 $\mu$m or more and small particles of less than 5 $\mu$m (i.e., droplet nuclei). Here, the smaller particles of less than 5$\mu$m have a slower falling speed, which is about 0.06 cm/s to 1.5 cm/s. Assuming the fall velocity of 0.06 cm/s, it would take about 27 minutes for a particle less than 5 $\mu$m to fall by one meter.

Therefore, the pause time Tb may be set to 25 minutes or less, for example. In this case, it makes it possible to appropriately irradiate small particles (i.e., droplet nuclei) of less than 5 $\mu$m, which tend to drift in the air, with UV light before they fall on the floor so as to effectively inactivate airborne viruses and bacteria. After the droplet nuclei have fallen to the ground, the accumulation of sediment (dust, etc.) around the viruses and bacteria may act as a barrier to the UV light. In contrast, by irradiating with UV light in the air with little amount of shielding, the bacteria and viruses can be expected to be reduced.

When the viruses are irradiated with UV light multiple times in the air, a pause time Tb of 10 minutes or less is preferable.

According to the present embodiment, the controller unit 20A may be configured to change the operation cycle of the lighting operation and the pause operation by the UV irradiation unit 10C in accordance with the proliferation situation of microorganisms and/or viruses harmful to humans or animals in the facility or vehicle. In this case, the conditions of the intermittent lighting (including the integrated light intensity per one lighting operation, the irradiance at the time of the lighting operation, the lighting time Ta, the pause time Tb, and the lighting duty ratio Td) may be individually and freely changeable, or the controller unit 20 may be configured to switch between a plurality of different pre-set operational modes.

For example, during periods when there is little or no human traffic, such as facility holiday periods or off-season periods, there is little need for frequent UV irradiation. Therefore, in such periods, the pause time Tb may be set to be longer.

On the other hand, for example, it can be determined to be a situation in which microorganisms and/or viruses harmful to the human bodies or animals tend to proliferate, if it is observed to be in a time zone of day in which there is a lot of human or animal traffic, a situation in which the proliferating environment of bacteria is easily established, or a situation in which an infectious disease is spreading. In such a situation, the operation cycle may be changed automatically or manually, for example, such that the pause time Tb becomes shorter. In the case of changing the operation cycle automatically, for example, the time of day, temperature, humidity, frequency of human or animal traffic, and other conditions may be detected by sensors, and the operation cycle may be changed by judging the proliferating situation based on the detected signals. On the other hand, in the case of manually changing the operation cycle, a signal indicating the operation mode selected by the user according to the proliferation status may be received, and the operation cycle may be changed based on the received signal.

According to the inactivation apparatus and the inactivation method of the present invention, it makes it possible to provide the inherent sterilization and virus inactivation capabilities of the ultraviolet light without adversely affecting the human body due to the UV light irradiation. In particular, unlike conventional UV light sources, taking advantage of the feature that it can be used in a manned environment, by installing the inactivation apparatus in an enclosed space in which humans or animals can enter and exit, it makes it possible to irradiate the entire interior of the enclosed space with the UV light so as to provide virus inhibition and sterilization of the air and surfaces of installed components in the enclosed space.

This addresses Goal 3 of the UN-led Sustainable Development Goals (SDGs): "Ensure healthy lives and promote well-being for all at all ages" and contributes significantly to Target 3.3 "By 2030, end the epidemics of AIDS, tuberculosis, malaria and neglected tropical diseases and combat hepatitis, water-borne diseases and other communicable diseases".

Although specific embodiments have been described above, the embodiments described are illustrative only and are not intended to limit the scope of the present invention. The apparatus and method described herein may be embodied in other forms than as described above. In addition, it is also possible to appropriately omit, substitute, or modify the above described embodiments without departing from the scope of the present invention. Embodiments with such omissions, substitutions and modifications fall within the scope of the appended claims and equivalents thereof and also fall within the technical scope of the present invention.

REFERENCE SIGNS LIST

10A, 10B and 10C: UV Irradiation Unit; 10D: Light Source for Illumination; 11: Human Detecting Sensor; 12: Pressure Sensor; 13: Door Sensor; 20: Controller Unit; 100: Inactivation Apparatus; 200: Enclosed Space (Private Toilet Room); 200A: Facility; 201: Ceiling; 202: Wall Portion; 203: Door; 300: Human

**Claims**

1.  An inactivation apparatus for inactivating microorganisms and/or viruses, comprising:

    an ultraviolet light irradiation unit configured to irradiate a surface or a space in a facility or a vehicle that a human or an animal is present with light including ultraviolet light having a wavelength that inactivates the microorganisms and/or viruses; and
    a controller unit configured to control irradiation and non-irradiation of the light by the ultraviolet light irradiation unit,
    the ultraviolet light included in the light emitted from the ultraviolet light irradiation unit being light having a wavelength range between 190 nm to 235 nm, and
    the controller unit controlling the ultraviolet light irradiation unit to alternately repeat a light-emitting operation and a non-light-emitting operation of the light based on the wavelength of the ultraviolet light included in the light irradiated from the ultraviolet light irradiation unit.

2.  The inactivation apparatus according to Claim 1, wherein
    the ultraviolet light irradiation unit emits ultraviolet light having a center wavelength between 200 nm and 230 nm.

3.  The inactivation apparatus according to Claim 1 or 2, wherein
    the ultraviolet light irradiation unit emits ultraviolet light having a center wavelength of 222 nm.

4.  The inactivation apparatus according to Claim 1 or 2, wherein
    the ultraviolet light irradiation unit emits ultraviolet light having a center wavelength of 207 nm.

5.  The inactivation apparatus according to any one of Claims 1 to 4, wherein
    the controller unit controls the ultraviolet light irradiation unit such that an integrated light intensity by a single light-emitting operation is to be equal to or less than 10 mJ/cm$^2$.

6.  The inactivation apparatus according to any one of Claims 1 to 5, wherein
    the controller unit controls the ultraviolet light irradiation unit such that an integrated light intensity by a single light-emitting operation is to be equal to or less than 5 mJ/cm$^2$.

7.  The inactivation apparatus according to any one of Claims 1 to 6, wherein
    the controller unit controls the ultraviolet light irradiation unit such that a single light-emitting operation time is to be equal to or less than 50% with respect to a sum of the single light-emitting operation time of the ultraviolet light irradiation unit and a single non-light-emitting operation time of the ultraviolet light irradiation unit.

8.  The inactivation apparatus according to any one of Claims 1 to 7, wherein
    the controller unit controls the ultraviolet light irradiation unit such that a single light-emitting operation time is to be equal to or less than 5% with respect to a sum of the single light-emitting operation time of the ultraviolet light irradiation unit and a single non-light-emitting operation time of the ultraviolet light irradiation unit.

9.  The inactivation apparatus according to any one of Claims 1 to 8, wherein
    the controller unit controls the ultraviolet light irradiation unit such that a single light-emitting operation time is to be equal to or greater than 1% of a sum of the single light-emitting operation time of the ultraviolet light irradiation unit and a single non-light-emitting operation time of the ultraviolet light irradiation unit.

10. The inactivation apparatus according to any one of Claims 1 to 9, wherein
    a single light-emitting operation time of the ultraviolet light irradiation unit is set to a time Ta (sec) satisfying a following formula:

$$Ta \leq D_{max} / (W*N),$$

    where a maximum allowable daily ultraviolet light exposure amount to the human body, which is determined based on to the wavelength of the ultraviolet light to be irradiated, is $D_{max}$ (mJ/cm$^2$), irradiance at an ultraviolet irradiated surface of the human body is W (mW/cm$^2$), and a number of times the light-emitting operation is performed per day is N.

**11.** The inactivation apparatus according to any one of Claims 1 to 10, wherein
a single light-emitting operation time of the ultraviolet light irradiation unit is set to be equal to or less than one minute.

**12.** The inactivation apparatus according to any one of Claims 1 to 11, wherein
the ultraviolet light irradiation unit includes a KrCl excimer lamp that emits ultraviolet light having a center wavelength of 222 nm.

**13.** The inactivation apparatus according to any one of Claims 1 to 11, wherein
the ultraviolet light irradiation unit includes a KrBr excimer lamp that emits ultraviolet light having a center wavelength of 207 nm.

**14.** The inactivation apparatus according to any one of Claims 1 to 13, wherein
the ultraviolet light irradiation unit includes a light emitting diode (LED) or a laser diode (LD) that emits ultraviolet light.

**15.** The inactivation apparatus according to any one of Claims 1 to 14, wherein
a single light-emitting operation time of the ultraviolet light irradiation unit is set to be equal to or less than one hour.

**16.** The inactivation apparatus according to any one of Claims 1 to 15, wherein
a single light-emitting operation time of the ultraviolet light irradiation unit is set to be equal to or less than 25 minutes.

**17.** The inactivation apparatus according to any one of Claims 1 to 16, wherein
a non-light-emitting operation time is set to be equal to or greater than 2 hours in at least a part of operation cycles of the light-emitting operation and the non-light-emitting operation of the ultraviolet light irradiation unit.

**18.** The inactivation apparatus according to any one of Claims 1 to 17, wherein
the controller unit controls the ultraviolet light irradiation unit to be capable of changing an operation cycle of the light-emitting operation and the non-light-emitting operation in accordance with a proliferation status of the microorganisms and/or viruses in the facility or the vehicle.

**19.** An inactivation method of inactivating microorganisms and/or viruses, comprising the steps of:

irradiating, by an ultraviolet light irradiation unit, a surface or a space in a facility or a vehicle that a human or an animal is present with light including ultraviolet light having a wavelength range between 190 nm to 235 nm as light including ultraviolet light having a wavelength that inactivates the microorganisms and/or viruses; and controlling irradiation and non-irradiation of the light by the ultraviolet light irradiation unit such that the ultraviolet light irradiation unit is controlled to alternately repeat a light-emitting operation and a non-light-emitting operation of the light based on the wavelength of the ultraviolet light included in the light irradiated from the ultraviolet light irradiation unit.

# FIG. 1

# FIG. 2

```
            ( START )
                │
    ┌───────────┤◄──────────────────────────┐
    │       ┌───┴───┐                        │
  No│   ╱IS HUMAN PRESENCE╲  ～ S1           │
    └──╲    DETECTED?     ╱                   │
         ╲──────┬──────╱                      │
                │ Yes                         │
         ┌──────┴──────────┐                  │
         │ START TIMER 1 COUNTING │ ～ S2      │
         └──────┬──────────┘                  │
    ┌───────────┤                             │
    │       ╱───┴───╲                         │
  No│   ╱IS PREDETERMINED╲  ～ S3             │
    └──╲  TIME T1 ELAPSED? ╱                  │
         ╲──────┬──────╱                      │
                │ Yes                         │
         ┌──────┴──────────┐                  │
         │ STOP TIMER 1 COUNTING │ ～ S4       │
         └──────┬──────────┘                  │
         ┌──────┴──────────┐                  │
         │ STOP UV IRRADIATION │ ～ S5         │
         └──────┬──────────┘                  │
    ┌───────────┤                             │
    │       ╱───┴───╲                         │
  No│   ╱IS HUMAN EXIT╲  ～ S6                │
    └──╲  DETECTED?   ╱                       │
         ╲──────┬──────╱                      │
                │ Yes                         │
         ┌──────┴──────────┐                  │
         │ START UV IRRADIATION │ ～ S7        │
         └──────┬──────────┘                  │
                └────────────────────────────┘
```

# FIG. 3

HUMAN DETECTING SENSOR

TIMER 1

UV IRRADIATION

T1

A          B

# FIG. 4

```
                    ┌─────────────┐
                    │    START    │
                    └──────┬──────┘
                           ▼
        ┌──────────────────────────────────┐
  No    │   IS HUMAN PRESENCE              │  S1
◄───────│      DETECTED?                   │
        └──────────────┬───────────────────┘
                       │ Yes
                       ▼
        ┌──────────────────────────────────┐
        │     START UV IRRADIATION          │  S11
        └──────────────┬───────────────────┘
                       ▼
        ┌──────────────────────────────────┐
        │   START TIMER 1 COUNTING          │  S2
        └──────────────┬───────────────────┘
                       ▼
        ┌──────────────────────────────────┐
  No    │   IS PREDETERMINED               │  S3
◄───────│    TIME T1 ELAPSED?              │
        └──────────────┬───────────────────┘
                       │ Yes
                       ▼
        ┌──────────────────────────────────┐
        │    STOP TIMER 1 COUNTING          │  S4
        └──────────────┬───────────────────┘
                       ▼
        ┌──────────────────────────────────┐
        │     STOP UV IRRADIATION           │  S5
        └──────────────┬───────────────────┘
                       ▼
        ┌──────────────────────────────────┐
  No    │    IS HUMAN EXIT                 │  S6
◄───────│     DETECTED?                    │
        └──────────────┬───────────────────┘
                       │ Yes
                       ▼
        ┌──────────────────────────────────┐
        │     START UV IRRADIATION           │  S7
        └──────────────┬───────────────────┘
                       ▼
        ┌──────────────────────────────────┐
        │   START TIMER 2 COUNTING          │  S12
        └──────────────┬───────────────────┘
                       ▼
        ┌──────────────────────────────────┐
  No    │   IS PREDETERMINED               │  S13
◄───────│    TIME T2 ELAPSED?              │
        └──────────────┬───────────────────┘
                       │ Yes
                       ▼
        ┌──────────────────────────────────┐
        │     STOP UV IRRADIATION           │  S14
        └──────────────────────────────────┘
```

# FIG. 5

# FIG. 6

```
                        ┌─────────────┐
                        │    START    │
                        └──────┬──────┘
                               ▼
        ┌──────────────────────────────────────────┐
        │         ╱ IS HUMAN PRESENCE ╲              │    S21
   No ──┤        ╲   DETECTED?        ╱              │
        │              │ Yes                         │
        │              ▼                             │
        │   ┌──────────────────────┐                │    S22
        │   │  STOP  UV IRRADIATION │                │
        │   └──────────┬───────────┘                │
        │              ▼                             │
        │         ╱ IS HUMAN SEATING ╲               │    S23
   No ──┤        ╲   DETECTED?        ╱              │
        │              │ Yes                         │
        │              ▼                             │
        │   ┌──────────────────────┐                │    S24
        │   │ START UV IRRADIATION  │                │
        │   └──────────┬───────────┘                │
        │              ▼                             │
        │   ┌──────────────────────┐                │    S25
        │   │ START TIMER 1 COUNTING│                │
        │   └──────────┬───────────┘                │
        │              ▼                             │
        │         ╱ IS PREDETERMINED ╲               │    S26
   No ──┤        ╲  TIME T1 ELAPSED? ╱              │
        │              │ Yes                         │
        │              ▼                             │
        │   ┌──────────────────────┐                │    S27
        │   │ STOP TIMER 1 COUNTING │                │
        │   └──────────┬───────────┘                │
        │              ▼                             │
        │   ┌──────────────────────┐                │    S28
        │   │  STOP UV IRRADIATION  │                │
        │   └──────────┬───────────┘                │
        │              ▼                             │
        │         ╱ IS HUMAN EXIT ╲                  │    S29
   No ──┤        ╲   DETECTED?     ╱                 │
        │              │ Yes                         │
        │              ▼                             │
        │   ┌──────────────────────┐                │    S30
        │   │ START UV IRRADIATION  │                │
        │   └──────────┬───────────┘                │
        └──────────────┴─────────────────────────────┘
```

# FIG. 7

HUMAN DETECTING SENSOR

PRESSURE SENSOR

TIMER 1

UV IRRADIATION

T1

P Q R

# FIG. 8

```
                    ┌─────────┐
                    │  START  │
                    └─────────┘
                         │
        No   ┌───────────────────────┐  S21
        ─────│ IS HUMAN PRESENCE      │
             │    DETECTED?           │
             └───────────────────────┘
                      │ Yes
        No   ┌───────────────────────┐  S23
        ─────│  IS HUMAN SEATING      │
             │    DETECTED?           │
             └───────────────────────┘
                      │ Yes
             ┌───────────────────────┐  S24
             │  START UV IRRADIATION  │
             └───────────────────────┘
                      │
             ┌───────────────────────┐  S25
             │ START TIMER 1 COUNTING │
             └───────────────────────┘
                      │
        No   ┌───────────────────────┐  S26
        ─────│  IS PREDETERMINED      │
             │   TIME T1 ELAPSED?     │
             └───────────────────────┘
                      │ Yes
             ┌───────────────────────┐  S27
             │  STOP TIMER 1 COUNTING │
             └───────────────────────┘
                      │
             ┌───────────────────────┐  S28
             │  STOP UV IRRADIATION   │
             └───────────────────────┘
                      │
        No   ┌───────────────────────┐  S29
        ─────│   IS HUMAN EXIT        │
             │    DETECTED?           │
             └───────────────────────┘
                      │ Yes
             ┌───────────────────────┐  S30
             │  START UV IRRADIATION  │
             └───────────────────────┘
                      │
             ┌───────────────────────┐  S31
             │ START TIMER 2 COUNTING │
             └───────────────────────┘
                      │
        No   ┌───────────────────────┐  S32
        ─────│  IS PREDETERMINED      │
             │   TIME T2 ELAPSED?     │
             └───────────────────────┘
                      │ Yes
             ┌───────────────────────┐  S33
             │  STOP UV IRRADIATION   │
             └───────────────────────┘
```

# FIG. 9

# FIG. 10

# FIG. 11

# FIG. 12

# FIG. 13

Wavelength (nm)

# FIG. 14

# FIG. 15

# FIG. 16

# FIG. 17

# FIG. 18

| Species | Dose for 3log reduction [ mJ／cm$^2$ ] | |
| --- | --- | --- |
| | 222 nm | 254 nm |
| MRSA | 15 | 10 |
| Pseudomonas aeruginosa | 8 | 4 |
| Escherichia. coli O157 | 9 | 5 |
| Pseudomonas aeruginosa | 10 | 4 |
| Bacillus Cereus | 44 | 90 |
| Bacillus subtilis | 30 | 60 |
| Clostridium difficile | 30 | 60 |
| MS2 | 23 | 50 |
| Feline Calicivirus | 24 | 24 |

# FIG. 19

# FIG. 20

# FIG. 21

# FIG. 22A

# FIG. 22B

# FIG. 23

# FIG. 24A

# FIG. 24B

1st      2nd      3rd      4th      5th

$5mJ/cm^2$ $10mJ/cm^2$ $15mJ/cm^2$ $20mJ/cm^2$ $25mJ/cm^2$

500s      500s

51m40s    51m40s

# FIG. 25

IRRADIATION AMOUNT ($mJ/cm^2$)

log SURVIVAL RATE

A2

C3

C4

# FIG. 26A

| 1st | 2nd | 3rd | 4th | 5th |
|-----|-----|-----|-----|-----|
| 1mJ/cm² | 2mJ/cm² | 3mJ/cm² | 4mJ/cm² | 5mJ/cm² |

10s          10s

50s          50s

# FIG. 26B

IRRADIATION AMOUNT (mJ/cm²)

A1

C5

log SURVIVAL RATE

<div align="center">

**INTERNATIONAL SEARCH REPORT**

</div>

| | International application No. |
|---|---|
| | PCT/JP2021/007746 |

**A. CLASSIFICATION OF SUBJECT MATTER**
Int. Cl. A47K17/00(2006.01)i, E03D9/00(2006.01)i, A61L2/10(2006.01)i, A61L9/20(2006.01)i
FI: A61L2/10, A47K17/00, E03D9/00 Z, A61L9/20
According to International Patent Classification (IPC) or to both national classification and IPC

**B. FIELDS SEARCHED**

Minimum documentation searched (classification system followed by classification symbols)
Int. Cl. A47K17/00, E03D9/00, A61L2/10, A61L9/20

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/186880 A1 (SUN ENERGY CORPORATION) 03 October 2019, claims 1, 6, paragraphs [0021], [0022], [0030], [0069], [0085], [0136], [0137], [0165] | 1-19 |
| Y | JP 2014-508612 A (THE TRUSTEES OF COLUMBIA UNIVERSITY IN THE CITY OF NEW YORK) 10 April 2014, paragraphs [0009], [0024] | 1-19 |
| A | US 2018/0339073 A1 (GENERAL ELECTRIC COMPANY) 29 November 2018, claims, fig. 1 | 1-19 |
| A | JP 2004-510118 A (HAMILTON BEACH PROCTOR SILEX INC.) 02 April 2004, paragraph [0045] | 1-19 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16.04.2021 | 27.04.2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2021/007746

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2017-528258 A (DAYLIGHT MEDICAL INC.) 28 September 2017, claims | 1-19 |

Form PCT/ISA/210 (continuation of second sheet) (January 2015)

**INTERNATIONAL SEARCH REPORT**

Information on patent family members

International application No.

PCT/JP2021/007746

| Patent Documents referred to in the Report | Publication Date | Patent Family | Publication Date |
|---|---|---|---|
| WO 2019/186880 A1 | 03.10.2019 | JP 6490318 B1 | |
| JP 2014-508612 A | 10.04.2014 | US 2015/0073396 A1 paragraphs [0009], [0033] US 2016/0107000 A1 US 2018/0169279 A1 US 2019/0160305 A1 US 2019/0381336 A1 US 2019/0388706 A1 US 2020/0085984 A1 US 2020/0215215 A1 WO 2012/122210 A1 EP 2683442 A1 | |
| US 2018/0339073 A1 | 29.11.2018 | US 2017/0151359 A1 US 2016/0271281 A1 WO 2016/149055 A1 EP 3270978 A1 TW 201707728 A CA 2978469 A CN 107427595 A MX 2017011933 A | |
| JP 2004-510118 A | 02.04.2004 | US 6494940 B1 column 7, lines 36-44 GB 2380954 A WO 2002/026349 A1 AU 1180602 A TW 504391 B CN 1462205 A MX PA03002377 A | |
| JP 2017-528258 A | 28.09.2017 | US 2016/0317690 A1 claims US 2018/0055964 A1 US 2019/0321504 A1 WO 2016/049143 A2 EP 3197505 A1 AU 2015320781 A AU 2018208755 A | |

Form PCT/ISA/210 (patent family annex) (January 2015)

**EP 3 957 222 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017528258 W

- US 20100032859 A

**Non-patent literature cited in the description**

- Ensure healthy lives and promote well-being for all at all ages. *UN-led Sustainable Development Goals (SDGs)*

- By 2030, end the epidemics of AIDS, tuberculosis, malaria and neglected tropical diseases and combat hepatitis, water-borne diseases and other communicable diseases. *Target 3.3*